# EUROPEAN PATENT SPECIFICATION

(11) **EP 1 320 754 B1**
(45) Date of publication and mention of the grant of the patent: **22.07.2009**
(21) Application number: 01969937.0
(22) Date of filing: 19.09.2001
(51) Int. Cl.: G01N 33/543

(54) **DETECTION OF PEPTIDES**
DETEKTION VON PEPTIDEN
DETECTION DE PEPTIDES

(30) Priority: 19.09.2000 GB 0022978
(43) Date of publication of application: 25.06.2003
(73) Proprietor: Millipore Corporation, Billerica, MA 01821 (US)
(72) Inventor: BARRY, Richard, Oxfordshire, OX14 4RY (GB); PLATT, Albert Edward, Abingdon, Oxfordshire OX14 4RY (GB); SCRIVENER, Elaine, Abingdon, Oxfordshire OX14 4RY (GB); SOLOVIEV, Mikhail, Abingdon, Oxfordshire OX14 4RY (GB); TERRETT, Johnathan Alexander, Abingdon, Oxfordshire OX14 4RY (GB)
(74) Representative: Chapman, Paul Gilmour
(86) International application number: PCT/GB2001/004182
(87) International publication number: WO 2002/025287

(56) References cited:
- WO-A-00/04389
- WO-A-00/54046
- WO-A-94/08597
- DE WILDT RUUD M T ET AL: "Antibody arrays for high-throughput screening of antibody-antigen interactions" NATURE BIOTECHNOLOGY, NATURE PUBLISHING, US, vol. 18, no. 9, September 2000 (2000-09), pages 989-994, XP002162316 ISSN: 1087-0156
- COTTRELL J S: "PROTEIN IDENTIFICATION BY PEPTIDE MASS FINGERPRINTING" PEPTIDE RESEARCH, NATICK, MA, US, vol. 7, no. 3, 1994, pages 115-124, XP002932021 ISSN: 1040-5704
- LI JIANJUN ET AL: "Application of microfluidic devices to proteomics research: Identification of trace-level protein digests and affinity capture of target peptides." MOLECULAR & CELLULAR PROTEOMICS, vol. 1, no. 2, February 2002 (2002-02), pages 157-168, XP002216222 February, 2002 ISSN: 1535-9476
- CAZARES L H ET AL: "DISCOVERY OF PROSTATE CANCER BIOMARKERS FROM LASER CAPTURE MICRODISSECTED (LCM) CELLS USING INNOVATIVE PROTEINCHIPTM SELDI MASS SPECTROSCOPY" PROCEEDINGS OF THE ANNUAL MEETING OF THE AMERICAN ASSOCIATION FOR CANCER RESEARCH, NEW YORK, NY, US, 2000, page 851 XP000929268 ISSN: 0197-016X

## Description

### Field of the Invention

This invention relates to affinity capture and detection of peptide fragments in a multi-dimension array.

### Background of the Invention

Monitoring the expressions and properties of a large number of proteins provides important information about the physiological or biochemical state of a cell. A cell can express a large number of different proteins, and the expression patterns (the number of proteins expressed and the expression levels) vary in different cell types, explaining why different cells perform different functions. Since many diseases associate with or even result from changes in protein expression pattern, comparing protein expression patterns between normal and disease conditions may reveal proteins whose changes are critical in the disease and thus identify proteins which are of therapeutic value or which aid diagnosis. Methods of detecting protein expression profiles also have important applications in, for example and without limitation, tissue typing, forensic identification, and clinical diagnosis.

Microarray formats for the quantitative detection of proteins (including antibody and antigen capture) have been developed for diagnosis (Ekins and Chu; Trends Biotechnol 1999, 17:217-8) and protein-protein interaction discovery (Walter et al. Curr Opin Microbiol 2000, 3:298-302; U.S. Patent No. 6,197,599). However, development of a microarray affinity capture system for semi-quantitative or quantitative proteomics similar to those used for semi-quantitative mRNA expression analyses (Schena et al. Trends Biotechnol. 1998, 16(7):301-6) has lagged for a number of reasons.

Firstly, there are few well-characterized, non-heterogeneous affinity capture agents (*e.g.,* DNA, protein, small molecule) exhibiting similar affinities for each target or analyte protein which a researcher might wish to detect. For mRNA analysis arrays the agents for affinity capture (cDNA clones and gene sequences, UniGene - http://www.ncbi.nlm.nih.gov/UniGene/) were designed and available well in advance of the microarray system (Southern, J. Mol Biol. 1975, 98:503-17, Maskos and Southern, Nucleic Acids Res 1992, 20:1679-84).

Secondly, the protein agents to be captured exist in a variety of highly heterogeneousnon-homogenous form, *e.g.,* small soluble proteins (cytokines), large, highly post-translationally modified proteins (*e.g.* collagen) and proteins with multiple domains of varying hydrophilicity (*e.g.* transmembrane receptors). Varying the pH in which the protein analyte is in a mixed population of heterogeneous protein types to correspond to, for example, subcellular location, may be required for folding. This means that any microarray affinity capture system for semi-quantitative or quantitative proteomics requires near impossible optimisation of capture affinity agent-protein interactions for a format to be achieved in which each affinity capture agent interacts with its corresponding target protein or family of target proteins in a uniform and predictable fashion. Alternatively, multiple arrays would be required to provide assay conditions specific for each different protein type such as those mentioned above.

Although mRNA microarrays are well advanced and identification of the differential sequences is straightforward, there is a large inconsistency between mRNA and protein levels (Gygi SP et al., Mol. Cell Biol. 1999, 19:1720-30) and since proteins are the primary agents responsible for disease and drug responses, predicting protein presence and amount by measurment of mRNA can lead to significant errors. Also current proteomics techniques such as 2D electrophoresis require complex procedures to identify differentially expressed proteins (Parekh et al. Journal of Commercial Biotechnology 2000, 6:284-291). A microarray format, high throughput system for quantitative direct detection of proteins such as is required in proteomics applications is thus highly desirable for, without limitation, diagnosis, pharmacoproteomics, identification of markers of disease and drug target discovery.

The present invention overcomes the deficiencies of current array-based proteomics techniques, and provides a system that permits qualitative and quantitative measurement of protein expression. These and other advantages of the invention are set forth more fully in the accompanying Description and Drawings.

### Summary of the Invention

An important aspect of the invention is a method for determining the presence of a protein in a sample. This method comprises submitting the sample to conditions that allow fragmentation of the protein into target peptide fragments. Following this, the target peptide fragments, which may be labeled to facilitate detection, are contacted with an array that comprises a solid support on which a plurality of capture agents, each designed to recognize a predicted a target peptide fragment according to a chosen fragmentation protocol of interest, are bound, *i.e.,* an array of antibodies on a solid support. The array may consist of capture agents selected to bind to one or more peptide fragments derived from each protein of interest. Preferably, a plurality of target peptide fragment/capture agent combinations is employed for each protein, thus increasing the confidence of an analytical signal response. Specific binding of the target peptide fragments with the capture agents is indicative of the presence of the corresponding protein in the sample. Such binding can be detected by detecting the optional label, or directly by probing for the presence of bound target peptide fragment by a highly sensitive technique such as, without limitation, mass spectrometry,

In a particular embodiment, this method further comprises determining the relative amount of one or more proteins in a sample by quantitating the amount of the corresponding bound target peptide fragments; This may be achieved by determining the amount of each target peptide fragment bound to the matrix relative to the amount of one or more bound target peptide fragments from other expressed protein(s) in the same sample whose expression levels are already known or known to be invariant

This application additionally describes methods for the derivation of large numbers of affinity capture agents that recognize and bind to peptides derived from proteins, preferably under uniform, reproducible, standard binding conditions. Peptides generated by enzymatic or non-enzymatic cleavage of proteins result in a mixture of molecular entities that contains species, which are more suitable for interaction with affinity capture agents than a mixture of proteins. For example, and without limitation, proteins contain many regions which cannot easily be accessed for binding to affinity capture agents: regions are shielded from external binding by glycosylation or other forms of post-translational modification; regions of high hydrophobicity such as membrane or transmembrane domains may bind with poor specificity; some binding sites arise as a result of 3-dimensional folding of non-contiguous segments of the protein. Fragmentation of such complex structures results in a plurality of smaller units (peptides) which are nevertheless representative of and unique to each fragmented protein. By selecting target peptide fragments which are unique to each protein of interest and further selecting those which can interact with capture agents in a predictable and uniform fashion it is possible to achieve an accuracy, precision and reproducibility in detection of target peptide fragments exceeding that attainable with proteins in an array format. Thus any proteins from any class can be assayed semi-quantitatively or quantitatively from any given starting material for example and without limitation, body fluid, whole cells and mixed cell types from organs and microbial.

The application additionally describes methods for design of the peptides used to derive the affinity capture agents. Also provided are methods for dispensing and immobilization of affinity capture agents in a microarray format; and for detection, quantitation and verification of the captured peptides. Furthermore, the identities of the peptides and thus the proteins responsible for differential signals are easily established.

In a preferred embodiment, the target peptide fragment has a sequence determined by enzymatic or chemical cleavage of a protein by subjecting a list of proteins of known structure or conceptually translated nucleotides to theoretical cleavage and predicting the resulting "*in silico*" or theoretical sequence. Alternatively, the target peptide fragment has a sequence determined by directly sequencing a peptide fragment produced by enzymatic or Chemical cleavage of a protein by methods well known to one skilled in the art.

Capture agents may be and preferably are, antibodies. In a specific embodiment all antibodies have a similar binding affinity for their respective target peptide fragment.

The target peptide fragment may comprise one or more sites for post-translational modification of the protein. The peptide compound also may comprise one or more post-translational modification functional groups. In a particular embodiment, the capture agent binds the target peptide fragment whether or not it has undergone post-translational modification.

Preferably capture agents in the array recognize target peptide fragments from multiple proteins whose expression levels best correlate with a physiological or biochemical state, for example, and without limitation, as determined by multivariate analysis of protein expression levels. This physiological or biochemical state may be a response, such as, without limitation, a response to a xenobiotic stress; a hyperplastic, cancerous, or metastatic state; an apoptotic, dysfunctional or diseased state; or a particular phenotype. Central nervous system dysfunctions or diseases, such as depression, schizophrenia, vascular dementia and other neurodegenerative conditions are particularly contemplated. Cancerous states, such as breast cancer or hepatoma, also are encompassed.

According to the present invention, the conditions that allow fragmentation of the protein into target peptide fragments preferably comprise contacting the sample with a proteolytic enzyme. In a preferred embodiment the cleavage pattern of the proteolytic enzyme is used to determine theoretical enzymatic cleavage of the protein, and the same enzyme is used to obtain sequences of target peptide fragments for use in selecting specific affinity capture agents.

In another embodiment affinity capture agents may be selected using solid phase presentation of either peptide compounds or target peptide fragments of interest.

The method for determining the presence of a protein in a sample may further comprise determining whether the target peptide fragment comprises a post-translational modification, for example and without limitation, using mass spectrometric analysis.

### Description of the Drawings

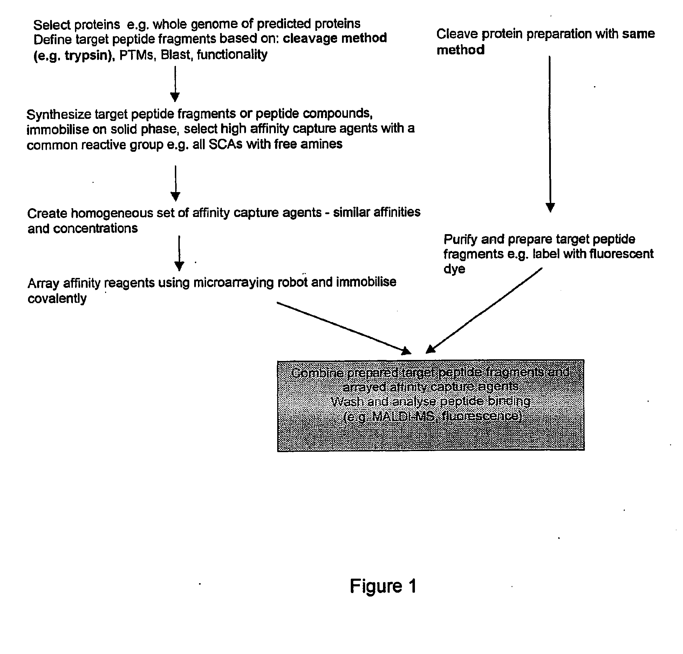

Figure 1 is a chart showing an example of a protocol for preparing an array and for the analysis of a protein preparation contacted with this array.
Figure 2 is the full protein sequence (SEQ ID NO:1) and chosen target peptide fragments for protein BCMP 11 (as detailed in Figure 1 of PCT Application No. WO 0163289 which is incorporated by reference in its entirety) when a trypsin-based cleavage system is to be employed. The two chosen peptide fragments (underlined) contain no Lys or Arg residues and are the most specific to BCMP 11 in this family of proteins.
Figure 3 is a MALDI-TOF mass spectrum showing the relative abundance of individual proteins present in ovary (A) and serum (B) samples captured by anti-albumin antibody. Protein extracts (1µg each) were incubated with identical arrays of antibodies immobilised on Hydrogel pads.
Figure 4 is a holder for mounting 2cm² MALDI targets. In this case it was used to mount a 2cm² silicon chip with immobilized aldehyde activated acrylamide containing proteins. This positions the surface of the 2cm² chip at the same height as the standard Perseptive Voyager gold and steel chip within the Mass Spectrometer (Perseptive.Voyager, Applied Biosystems, Framingham, MA)
Figure 5 is a MALDI-TOF mass spectrum showing desorption of albumin from a polyacrylamide pad.
Figure 6 is a MALDI-TOFmass spectrum showing desorption/ionization of EGF peptide.
Figure 7 shows the results of microarrays of antibodies incubated with a range of fluorescently-labeled human proteins; the protein labels indicated in the lower panel denote the binding specificity of the corresponding antibodies.
Figure 8 is a MALDI-TOF mass spectrum resulting from peptides derived from a crudetryptic digest of human serum albumin. Triangles denote peptides which were captured by Immobilized anti-albumin antibodies see Figure 9.
Figure 9 is a MALDI-TOF mass spectrum showing specific capture of three peptides (denoted by the triangles in Figure 8), derived from the a tryptic digest of human serum albumin, by the polyclonal anti-albumin antibody immobilized on hydrogel pads.

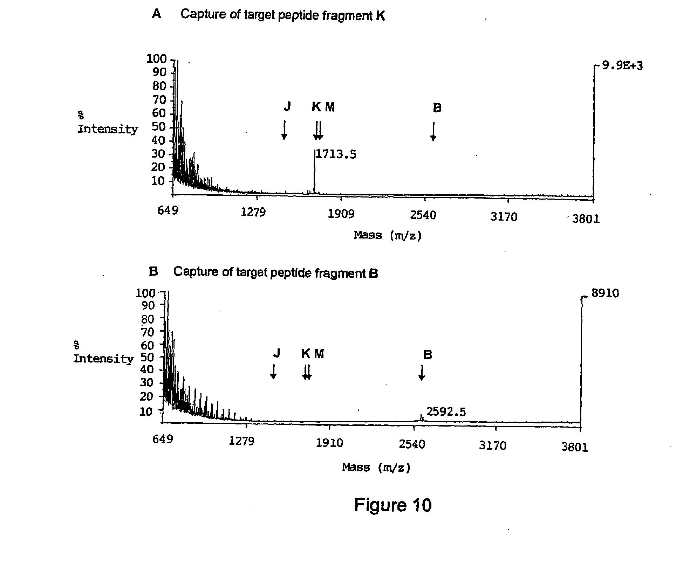

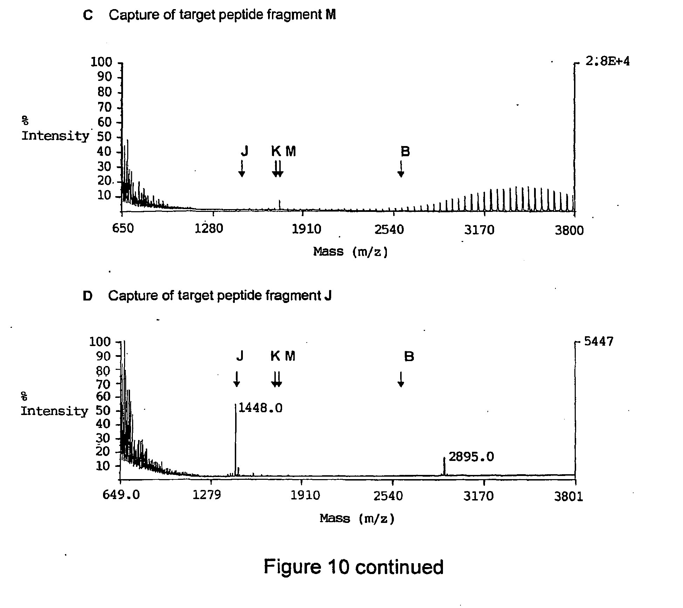

Figure 10 shows MALDI-TOF mass spectra identifying individual target peptide fragments captured from a mixture of VCAM peptides, individual short chain antibodies, each specific to target peptide fragments K, B, M and J were immobilised on separate 2 mm diameter Hydrogel pads. Panels A, B, C and D show specific capture of the expected target peptide fragment only. Arrows In each panel indicate expected masses of all of the four target peptide fragments in mixtures. In panel D, the higher mass peak corresponds to a dimer of the target peptide fragment 4, as is often seen for intense species in MALDI TOF MS. In spectrum C the higher mass peaks are due to polyethylene glycol which was present In the TBS buffer.

### Detailed Description

The present invention greatly advances the effort to develop proteomics microarrays. The microarrays of the invention reduce the complexity of protein binding assays by providing a uniform, standardized binding system based on interactions of capture agents (as defined below) with peptides. Peptides bind to binding partners (capture agents in the practice of the present invention) with relatively uniform kinetics and affinity (with some variation due to amino acid sequence), whether those binding partners are other peptides, antibodies, receptors, proteins, and even nucleic acids. In this respect, the microarrays of the invention can have binding features more like those associated with nucleic,acid hybridization arrays, and thus provide robust, standardized systems for detecting and, optionally, quantifying the total amount of a particular protein present. This is possible because the arrays of the invention are designed to detect peptides derived from cellular proteins, thus avoiding binding complexities of proteins.

One unique feature of this invention, then, is that the methods employ capture agents that specifically bind to peptides rather than proteins. Another feature of the methods of the invention lies in the ability to quantitate proteins of very different biophysical activities by detecting peptides, which have much more uniform biophysical activities. Again, use of peptide- (rather than protein-) specific capture agents facilitates this advantage.
The methods of the invention are useful for proteomics, pharmacoproteomics, identification of markers of disease, drug target discovery, diagnostics, and in conjunction with therapy.

### Definitions

**a)** The term "capture agent" means any compound that is capable of interacting eg. binding to a peptide compound or a target peptide fragment. The interaction should be as specific as possible, *i.e.,* without substantial cross-reaction with other peptides or different proteins usually present in a biological sample. High affinity capture agents, e.g., with a K_{eff} of 10⁻⁶ M⁻¹ or better or more preferably 10⁻⁷ M⁻¹ or better, or still more preferably 10⁻⁸ M⁻¹ or better are preferred. An agent that "recognizes" a peptide compound or a sequence thereof refers to the ability of this agent to specifically bind to this peptide compound. Exemplary capture agents include, without limitation, antibodies, receptors, proteins and binding domains thereof, nucleic acids, carbohydrates, lectins, and the like. Antibodies are the preferred capture agents because of their well characterized, uniform antigen binding properties and the relatively high affinity of binding.
**b)** The term "diagnosis" refers to the measurement or monitoring of protein markers of disease presence, predisposition or progression in an animal and most particularly a human, characterizing, selecting animals or humans for study, including participants in preclinical and clinical trials, and identifying those at risk for, or having a particular disorder, or those most likely to respond to a particular therapeutic treatment, or for assessing or monitoring an animal or human response to a particular therapeutic or drug treatment
**c)** The term "peptide compound" is used in its broadest sense to refer to a compound of two or more subunit amino acids, amino acid analogs or peptidomimetics. Preferably, for ease in synthesis, the peptide compound is 6 to about 25 amino acid residues in length (or the equivalent thereof of peptidomimetic subunits). The subunits may be linked by peptide bonds. In another embodiment, the subunit may be linked by other the bonds, *e.g.,* ester, ether, etc. As used herein the term "amino acid" refers to either natural and/or unnatural or synthetic amino acids, including glycine and both the D or L optical isomers, and amino acid analogs and peptidomimetics. Thus, peptide compounds of the invention may comprise D-amino acids, a combination of D- and L-amino acids, and various "designer" amino acids (*e.g.,* beta-methyl amino acids, C-alpha-methyl amino acids, and N-alpha-methyl amino acids, etc.). Additionally, by assigning specific amino acids at specific coupling steps, peptides with alpha-helices, beta-turns, beta-sheets, alpha-turns and cyclic peptide compounds can be generated. A peptide of three or more amino acids is commonly called an oligopeptide or peptide if the peptide chain is short e.g. less than about 30 amino acids. If the peptide chain is long, the peptide is commonly called a polypeptide or a protein.
   A peptide compound may be a target peptide fragment or may comprise the sequence or part of the sequence of a target peptide fragment The binding of a peptide compound is preferably indistinguishable from that of the capture agent to the target peptide fragment. Preferably, the peptide compound comprises the part of the sequence of the target peptide fragment that uniquely identifies the protein comprising that sequence. Typically, peptide compounds are generated by chemical synthesis, *e.g.,* solid phase peptide synthesis. However, peptide compounds can also be obtained by purifying the target peptide fragment or a subfragment thereof. Peptide compounds may be derivatized to facilitate their use, which includes generating capture agents and selecting capture agents. For example, the peptide compound may be derivatized to correspond to a post-translational modification of the protein or target peptide fragment In addition, the peptide compound may contain a linker group or functional reagent for binding to a carrier molecule for immunization, or a solid support for screening for affinity capture agents that are specific for the peptide compound.
**d)** The term "protein of interest" means a protein, the detection of which is desired and that comprises a protein of known sequence or a protein whose sequence is at least partly known or is a conceptual translation of a nucleotide sequence considered to encode a protein or peptide.
   Proteins of interest are not limited in accordance with the present invention, and thus include secreted proteins, integral membrane proteins (including receptors, cell adhesion molecules, and the like), cytoplasmic proteins, proteins from complexes (*e.g.,* ribosomal proteins, polymerase proteins, intracellular signal proteins, etc.), organelle proteins (*e.g.,* mitochondrial proteins, lysosomal proteins, nuclear proteins, endoplasmic reticulum proteins, etc., whether or not membrane associated), and nucleic acid binding proteins (*e.g.,* histones, repressors, transcriptional activators, trans-acting enhancer factors, ribonucler proteins, etc.). As noted above, an advantage of the invention lies in the detection of peptide fragments of a protein of interest, which reduces or eliminates competitive interactions and anomalous binding resulting from endogenous protein characteristics.
**e)** As used herein, a "protein of known sequence" is any protein including, without limitation, a protein in a sample or body fluid or in a cell or tissue for which partial or full sequence information is available. Such sequence information can be available from any source, including, but not limited to, traditional biochemistry, genomics, functional genomics, proteomics analysis, and the like, Preferred methods for identifying a protein of known sequence are proteomics approaches, which are well known in the art. The proteomics approach has the advantage of correlating the presence of actual protein within a cell of interest to, for example, the physiological state of the cell of interest In contrast, functional genomics, which measures messenger RNA levels, has a (less direct correlation with protein expression and thus with the physiological or biochemical status of the cell.
**f)** "Proteomics analysis" using the microarrays described in the invention can be used to determine the physiological or biochemical state of a body fluid, a tissue or a cell. The or physiological or biochemical state refers to the condition of a cell or tissue after it subjected to a stimulus or is contacted with a molecule, such as a drug, hormone, or other ligand that stimulates or effects cellular activity, after the cell or tissue is partially or completely transformed to become for example, but not limited to, hyperplastic, cancerous, or metastatic, where the cell has entered an apoptotic or other pathway, whether the cell is dysfunctional or diseased, and the type of the cell, *i.e.,* the tissue from which the cell is derived. All of this information is available from the proteomics analysis. Proteomics analysis can also be used to determine the protein complement of body fluids or exudates.
**g)** The term "specifically recognizes" as used herein refers to a capture agent that preferentially binds to its cognate target peptide fragment to a greater degree i.e. with greater affinity than any other molecule, such as the full length protein.
**h)** A "target peptide fragment" comprises an identifiable sequence from the protein of interest Preferably, a target peptide fragment of a protein of known sequence is produced by a reproducible, sequence specific proteolysis. For example, without limitation, many proteolytic enzymes such as, without limitation, endopeptidases, cleave proteins at known cleavage cites. Such enzymes are particularly useful for generating target peptide fragments in accordance with the present invention. The target peptide .. fragments and proteolytic enzymes for generating them, as well as other approaches for generating target peptide fragments, are discussed in greater detail *infra.* Target peptides fragments can also be produced by chemical proteolysis. A combination of one or more target peptide fragments is representative of the protein from which the target peptide fragments are derived and is indistinguishable from other target peptide fragments derived from other proteins.

The present invention represents an advance in efforts to develop proteomics analysis. The inventors have discovered that it is possible to assay for the presence of peptide fragments derived from proteins rather than the entire, intact protein and that such assays can be performed with a level of consistency which is largely independent of the nature or origin of the protein of interest. This allows a semi-quantitative or more preferably a quantitative analysis to be performed. Such an approach has a number of advantages. Firstly, it is consistent with approaches for determining the identity of a protein present in a biological sample by two-dimensional electrophoresis and mass spectrometry (see US patents 6,064,754 and 6,27.8,794 and in WO-A-02/21139 filed on 10th September 2001 ). Mass spectrometric data are readily obtained from peptides derived by proteolysis of proteins isolated by two-dimensional electrophoresis. The second advantage of the invention is that affinity capture agent-target peptide fragment interactions are better understood, more predictable and more readily controlled than affinity capture agent-protein interactions. In particular antibody-peptide interactions are well understood. By nature of their small size, target peptide fragments contacted with an array are well-exposed to the affinity capture agents present on that array. In contrast, proteins may be subject to degeneration, misfolding, post-translational modification, and other environmental influences that may result in adoption of a conformation that reduces or blocks binding to antibody or results in unpredictable binding, whether by increasing or decreasing it.

The invention further provides for generating affinity capture agents, such as antibodies, that are highly specific for unique sequences within the protein of interest. Using such a highly specific approach substantially diminishes cross reactivity which can be problematic when proteins of interest are related molecules.

Finally, the peptide approach allows redundancy in quantitating the amount of protein present in a sample since it is possible to use a plurality of affinity capture agents each specific to one of a plurality of target peptide fragments derived from the same protein of interest. This results in a plurality of binding signals or responses being obtained for each protein of interest being analysed; a plurality of target peptide fragments used to generate the affinity capture agents will enable a more accurate quantitation of the protein of interest present in the sample by internal comparison of the detection signals from each target peptide fragment for any given protein of interest.

In short, the present invention increases the power of a proteomics microarray and simplifies this approach technically. The examples, *infra,* clearly demonstrate that the feasibility of quantitative protein detection using peptide specific capture agent arrays as set forth in the invention.

In a preferred embodiment, the invention permits generating capture agents against target peptide fragments of proteins of known sequence from genomic, functional genomic, or proteomics analysis. Thus, the capture agent microarray can be used as a broad proteomics analytical tool, e.g., for analyzing protein expression, post translational modification, or other cell or tissue physiological characteristics. In a specific embodiment, the invention permits performing multivariate analysis of levels of individual protein expression of all, most all, or a large number of proteins of interest in the cell.. Because of the specificity conferred by the capture agents for target peptide fragments, including without limitation, especially post-translational modifications, combined with the prior knowledge of the sequence of the protein of interest and thus the identity of the protein from which any peptide is derived, a general capture agent microarray of this sort provides a powerful tool for proteomics analysis to complement traditional two-dimensional electrophoresis.

In another preferred embodiment, proteins of interest of known sequence that have a high correlation with the physiological or biochemical status of the cell or tissue are selected for producing capture agents for diagnosis for use in monitoring arrays, and for therapeutic purposes in accordance with the invention. Such proteins can be identified using multivariate analysis of levels of expression of all, most all, or a large number of proteins of interest in the cell, either by traditional biochemical approaches, two-dimensional gel proteomics analysis, or by using a microarray of the invention. For example, of the thousands of proteins in a cell, two hundred to three hundred proteins may be identified by traditional two-dimensional gel electrophoresis. Of these, 5 or more, 10 or more, particularly 20 or more, and more particularly still 30, 40 or 50 or more, such proteins may be the most significant indicators of the physiological or biochemical state i.e. "marker proteins". Accordingly, to maximize efficiency, an array of the invention can comprise capture agents for target peptide fragments from each of these 5 or more proteins of known sequence. More preferably, the array will comprise two or more capture agents specific for two or more unique target peptide fragments from each protein of interest of known sequence of primary interest.

As described herein, preferably, to enhance the accuracy and reproducibility of the system, especially for more effective quantitation, the binding affinity of each capture agent for its target peptide fragment is similar to that of the other capture agents for their respective target peptide fragments. Similar binding affinities mean that the variance of binding affinities among all of the capture agents for their corresponding target peptide fragment is within 100-fold, and preferably within 10-fold. Binding affinities within a 10-fold range will demonstrate similar binding characteristics in an assay and result in a more accurate semi-quantitative or quantitative method. Methods of detecting full length conformationally correct biologically active proteins using affinity-based capture agents in array formats are complicated by the fact that many sites of potential binding between the protein of interest and the affinity capture agent are obscured or inaccessible in the full length folded protein. In contrast each protein yields at least one or more target peptide fragments. As long as at least one such target peptide fragment is available from each protein of interest and is specific to each protein of interest and a capture agent binds with sufficient affinity to such target peptide fragments, the method of the invention will produce an assay system capable of detecting any protein of whatever biological, biochemical and biophysiological characteristic under a set of assay conditions which are largely independent of the characteristics of any protein of interest.

The present invention contemplates pre-identification of proteins of know sequence. However, the arrays of the invention can be used to further refine the set of proteins of known sequence to be used in diagnosis, monitoring, and therapeutic indications. Thus, using the invention disclosed herein allows production of an array comprising capture agents for target peptide fragments from, for example, greater than 100 candidate proteins (fewer can also be evaluated with great advantage). Based on the quantititive data available from the arrays of the invention, it will be possible to specifically identify those proteins of interest from the cell or tissue that, as set forth above, provide the most information about the biochemical or physiological status of the cell or tissue.

Once proteins of known sequence are identified, it is then possible to design peptide compounds, prepare capture agents, prepare arrays comprising capture agents preferentially immobilized on a solid support, and test for the presence, and optionally, the amount of target peptide fragments from the proteins of interest.

### Design of Peptide Compounds for Preparation of Capture Agents

The peptide compounds used in the method of the invention may be designed by an *in silico* analysis of anticipated cleavage sites of a protein of interest. This *in silico* cleavage may be performed on:
- protein sequences that have been compiled using mass spectrometry or Edman analysis of a sample
- protein sequences noted in databases such as SwissProt (World Wide Web at ca.expasy.org/sprot/)
- protein sequences obtained by conceptual translation of nucleotide databases such as the EMBL nucleotide sequence database (World Wide Web at www.ebi.ac.uk/embl/index.html). In a preferred embodiment enzymatic (preferably an endopeptidase such a trypsin) or chemical (eg cyanogen bromide) cleavage patterns of proteins are used; these are readily predictable and reproducible (Chong et al. Rapid Commun Mass Spectrom 1998, 12(24):1986-93). The number of target peptide fragments selected for each protein of interest is at least one.

Such proteins of interest may be proteins previously identified for example in disease association studies, as described in greater detail below. They may also be proteins that are not known to be associated with diseases but may instead reflect the activation state or cycle or functionality, or phenotype or some other characteristic of the cell, any of which may be determined by the methods of the invention.

In one preferred embodiment, a listing of real peptides that have been detected by mass spectrometry in proteomics experiments (as described in US patent 6,064,754 and WO-A-02/21139, filed one 10^{th} September 2001) is constructed. According to this embodiment, target peptide fragments are selected from this listing, or subsets thereof. Furthermore this listing provides relative analytical detection intensities for each target peptide fragment within the plurality of target peptide fragments from each protein of interest giving greater accuracy and precision of detection and improved quantitation. In a further preferred embodiment the proteins of interest are selected from proteins present in an experimentally observed proteime. Such a proteome can be generated by conducting two-dimensional gel experiments, with mass spectrometry analysis, using the same reference technologies referred to herein,

In another embodiment, the human genome is scanned beforehand with an exon-predicting software to extract DNA data most likely to represent expressed proteins. Examples of such software programs are: GenScan (Burge and Karlin J. Mol. Biol. 1997, 268:78-94; genes.mit.edu/GENSCAN.html on the World Wide Web) and GeneFinder (searchlauncher.bcm.tmc.edu/gene-finder/gfb.html on the World Wide Web). The extracted DNA sequences are subsequently translated into protein sequences that are in turn conceptually cleaved by any chosen method of proteolysis to provide target peptide fragments.

In a preferred embodiment the selection of target peptide fragments is based on sequences from the human genome that are known to unambiguously code for proteins, i.e. exons, through direct protein analysis. This provides a substantial improvement in the signal-to-noise ratio of the array detection by avoiding the possibility of non-specific binding of real peptides to antibodies raised against conceptual translations of noncoding human genome sequences.

In yet another embodiment, known recognition motifs or specific determinants within a protein of interest are used to select a sequence for preparing a capture agent, including without limitation known domain-peptide fragment or domain-domain interaction modules. Such interaction modules and specificity determinants could include, but are not limited to, concensus sequences determining the specificity of protein kinases and phosphatases, calmodulin recognition motifs (Rhoads and Friedberg, 1997, FASEB 11:331-340), LG/LNS domains (RUdenko et al., 2001, TIBS 26:363-368), zinc finger domains (Gillooly, et al., 2001, Biochem. J. 355:249-258), RING domains (Borden, 2000, J. Mol. Biol. 295: 1103-1112), SH3 and SH2 domains, EH, PDZ,SAMS, FYVE and PH domains and others as mentioned in Pawson and Nash, 2000, Genes Dev. 14:1027-1047(see also Schultz et al., 2000 Nucleic Acids Res. 28:231-234; World Wide Web at smart.embl.Heidelberg.de/)

One skilled in the art can also identify sequence information from peptides analyzed by mass spectrometry and/or tandem mass spectrometry using various spectral interpretation methods and database searching tools. Examples of some of these methods and tools can be found at the Swiss Institute of Bioinformatics web site on the World Wide Web at www.expasy.com, and the European Molecular Biology Laboratory web site on the World Wide Web at www.mann.embl-heidelberg.de/Services/PeptideSearch/.

In a specific embodiment, an array is constructed in which peptide fragments are arrayed and used as capture agents. The array can then be exposed to a sample containing one or more proteins of interest or a plurality of target peptide fragments from such proteins of interest to identify sequences in the protein of interest or its corresponding target peptide fragments which bind to the arrayed peptide fragments. This identifies arrayed peptide fragments of use as capture agents for specific target peptide fragments and also identifies peptide-peptide binding of potential biological or physiological significance. In a further specific embodiment peptide fragments produced from a plurality of proteins, *e.g.,* peptides identified by a plurality of exon-mapped sequences, may be used as peptide compounds. The compounds can be used to produce arrays of capture agents that are then contacted with a test sample and a control. Any difference in signals between the test sample and the control sample indicates for a given capture agent that such a capture agent or plurality of capture agents is an effective tool for monitoring changes in such a sample.

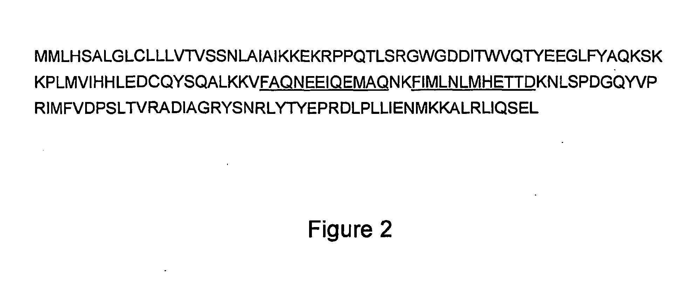

The preferred method is to design synthetic peptides as peptide compounds for selecting the capture agents with the required binding specificity. The peptide compounds defined by this invention for use in designing capture agents can be designed individually according to appropriate methods but should in general correspond to the predicted peptide sequences or part thereof, according to the chosen digestion reagents which will be used in the detection of experimental peptides derived from a protein of interest. The number of peptide fragments selected for each protein as target peptide fragments can be one and more preferably more than one. An example of the selection of such target peptide fragments from the protein BCMP 11 (SEQ ID no. 1) is shown in figure 2. The chosen peptides should also preferably be unique to the proteins being assayed, as determined for example and without limitation by means of the Blast algorithm (Altschul, et. al, J. Mol. Biol, 1990, 215:403-410) or other techniques known to one skilled in the art. Signal sequence peptides which are removed from the immature protein are also preferably avoided.

Furthermore, target peptide fragments can be selected based on information regarding whether they may be present in a protein of interest in a region subject to post-translational modifications and subject to such post-translational modifications. Such peptides may be used to design arrays useful to diagnose a disease associated with such specific post-translational modifications. This information can come directly from the literature (*e.g.* Hoffman et. al, Biochemistry, 1996,35(47):14849-61.3), from direct evidence (*e.g.,* tandem MS) or from prediction *(e.g.* intracellular tyrosines are often subject to phosphorylation, PSORTII on the World Wide Web).

### Post-translational Modifications

Over 250 post-translational modifications have been described (See the World Wide Web at pir.georgetown.edu/cgi-bin/pirwww/nbrfcg?db=R [retrieved on 2000-11-22]; also see Barker et al. Nucleic Acids Research 2000, 28:41-44, the contents of which are hereby incorporated by reference in their entirety).

Examples of post-translational modifications that may be identified, sequenced or mapped using the methods described herein, include but are not limited to: N-formyl-L-methionine; L-selenocysteine; L-cystine; L-erythro-beta-hydroxyasparagine; L-erythro-beta-hydroxyaspartic acid; 5-hydroxy-L-lysine; 3-hydroxy-L-proline; 4-hydroxy-L-proline; 2-pyrrolidone-5-carboxylic acid; L-gamma-carboxyglutamic acid; L-aspartic 4-phosphoric anhydride; S-phospho-L-cysteine; 1'-phospho-L-histidine; 3'-phospho-L-histidine; O-phospho-L-serine; O-phospho-L-threonine; 04'-phospho-L-tyrosine; 2'-[3-carboxamido-3-(trimethylammonio)propyl]-L-histidine; N-acetyl-L-alanine; N-acetyl-L-aspartic acid; N-acetyl-L-cysteine; N-acetyl-L-glutamic acid; N-acetyl-L-glutamine; N-acetylglycine; N-acetyl-L-isoleucine; N2-acetyl-L-lysine; N-acetyl-L-methionine; N-acetyl-L-proline; N-acetyl-L-serine; N-acetyl-L-threonine; N-acetyl-L-tyrosine; N-acetyl-L-valine; N6-acetyl-L-lysine; S-acetyl-L-cysteine; N-formylglycine; D-glucuronyl-N-glycine; N-myristoyl-glycine; N-palmitoyl-L-cysteine; N-methyl-L-alanine; N,N,N-trimethyl-L-alanine; N-methylglycine; N-methyl-L-methionine; N-methyl-L-phenylalanine; N,N-dimethyl-L-proline; omega-N,omega-N'-dimethyl-L-arginine; omega-N,omega-N-dimethyl-L-arginine; omega-N-methyl-L-arginine; N4-methyl-L-asparagine; N5-methyl-L-glutamine; L-glutamic acid 5-methyl ester; 3'-methyl-L-histidine; N6,N6,N6-trimethyl-L-lysine; N6,N6-dimethyl-L-lysine; N6-methyl-L-lysine; N6-palmitoyl-L-lysine; N6-myristoyl-L-lysine; O-palmitoyl-L-threonine; O-palmitoyl-L-serine; L-alanine amide; L-arginine amide; L-asparagine amide; L-aspartic acid 1-amide; L-cysteine amide; L-glutamine amide; L-glutamic acid 1-amide; glycine amide; L-histidine amide; L-isoleucine amide; L-leucine amide; L-lysine amide; L-methionine amide; L-phenylalanine amide; L-proline amide; L-serine amide; L-threonine amide; L-tryptophan amide; L-tyrosine amide; L-valine amide; L-cysteine methyl disulfide; S-farnesyl-L-cysteine; S-12-hydroxyfarnesyl-L-cysteine; S-geranylgeranyl-L-cysteine; L-cysteine methyl ester; S-palmitoyl-L-cysteine; S-diacylglycerol-L-cysteine; S-(L-isoglutamyl)-L-cysteine; 2'-(S-L-cysteinyl)-L-histidine; L-lanthionine; meso-lanthionine; 3-methyl-L-lanthionine; 3'-(S-L-cysteinyl)-L-tyrosine; N6-carboxy-L-lysine; N6-1-carboxyethyl-L-lysine; N6-(4-amino-2-hydroxybutyl)-L-lysine; N6-biotinyl-L-lysine; N6-lipoyl-L-lysine; N6-pyridoxal phosphate-L-lysine; N6-retinal-L-lysine; L-allysine; L-lysinoalanine; N6-(L-isoglutamyl)-L-lysine; N6-glycyl-L-lysine; N-(L-isoaspartyl)-glycine; pyruvic acid; L-3-phenyllactic acid; 2-oxobutanoic acid; N2-succinyl-L-tryptophan; S-phycocyanobilin-L-cysteine; S-phycoerythrobilin-L-cysteine; S-phytochromobilin-L-cysteine; heme-bis-L-cysteine; heme-L-cysteine; tetrakis-L-cysteinyl iron; tetrakis-L-cysteiny) diiron disulfide; tris-L-cysteinyl triiron trisulfide; tris-L-cysteinyl triiron tetrasulfide; tetrakis-L-cysteinyl tetrairon tetrasulfide; L-cysteinyl homocitryl molybdenum-heptairon-nonasulfide; L-cysteinyl molybdopterin; S-(8alpha-FAD)-L-cysteine; 3'-(8alpha-FAD)-L-histidine; 04'-(8alpha-FAD)-L-tyrosine; L-3',4'-dihydroxyphenylalanine; L-2',4',5'-topaquinone; L-tryptophyl quinone; 4'-(L-tryptophan)-L-tryptophyl quinone; O-phosphopantetheine-L-serine; N4-glycosyl-L-asparagine; S-glycosyl-L-cysteine; 05-glycosyl-L-hydroxylysine; O-glycosyl-L-serine; O-glycosyl-L-threonine; 1'-glycosyl-L-tryptophan; 04'-glycosyl-L-tyrosine; N-asparaginyl-glycosylphosphatidylinositolethanolamine; N-aspartyl-glycosylphosphatidylinositolethanolamine; N-cysteinyl-glycosylphosphatidylinositolethanolamine; N-glycyl-glycosylphosphatidylinositolethanolamine; N-seryl-glycosylphosphatidylinositolethanolamine; N-alanyl-glycosylphosphatidylinositolethanolamine; N-seryl-glycosylsphingolipidinositolethanolamine; O-(phosphoribosyl dephospho-coenzyme A)-L-serine; omega-N-(ADP-ribosyl)-L-arginine; S-(ADP-ribosyl)-L-cysteine; L-glutamyl 5-glycerylphosphorylethanolamine; S-sulfo-L-cysteine; O4'-sulfo-L-tyrosine; L-bromohistidine; L-2'-bromophenylalanine; L-3'-bromophenylalanine; L-4'-bromophenylalanine; 3',3",5'-triiodo-L-thyronine; L-thyroxine; L-6'-bromotryptophan; dehydroalanine; (Z)-dehydrobutyrine; dehydrotyrosine; L-seryl-5-imidazolinone glycine; L-3-oxoalanine; lactic acid; L-alanyl-5-imidazolinone glycine; L-cysteinyl-5-imidazolinone glycine; D-alanine; D-allo-isoleucine; D-methionine; D-phenylalanine; D-serine; D-asparagine; D-leucine; D-tryptophan; L-isoglutamyl-polyglycine; L-isoglutamyl-polyglutamic acid; O4'-(phospho-5'-adenosine)-L-tyrosine; S-(2-aminovinyl)-D-cysteine; L-cysteine sulfenic acid; S-glycyl-L-cysteine; S-4-hydroxycinnamyl-L-cysteine; chondroitin sulfate D-glucuronyl-D-galactosyl-D-galactosyl-D-xylosyl-L-serine; dermatan 4-sulfate D-glucuronyl-D-galactosyl-D-galactosyl-D-xylosyl-L-serine; heparan sulfate D-glucuronyl-D-galactosyl-D-galactosyl-D-xylosyl-L-serine; N6-formyl-L-lysine; O4-glycosyl-L-hydroxyproline; O-(phospho-5'-RNA)-L-serine; L-citrulline; 4-hydroxy-L-arginine; N-(L-isoaspartyl)-L-cysteine; 2'-alpha-mannosyl-L-tryptophan; N6-mureinyl-L-lysine; 1-chondroitin sulfate-L-aspartic acid ester; S-(6-FMN)-L-cysteine; 1'-(8alpha-FAD)-L-histidine; omega-N-phospho-L-arginine; S-diphytanylglycerol diether-L-cysteine; alpha-1-microglobulin-Ig alpha complex chromophore; bis-L-cysteinyl bis-L-histidino diiron disulfide; hexakis-L-cysteinyl hexairon hexasulfide; N6-(phospho-5'-adenosine)-L-lysine; N6-(phospho-5'-guanosine)-L-lysine; L-cysteine glutathione disulfide; S-nitrosyl-L-cysteine; N4-(ADP-ribosyl)-L-asparagine; L-beta-methylthioaspartic acid; 5'-(N6-L-lysine)-L-topaquinone; S-methyl-L-cysteine; 4-hydroxy-L-lysine; N4-hydroxymethyl-L-asparagine; O-(ADP-ribosyl)-L-serine; L-cysteine oxazolecarboxylic acid; L-cysteine oxazolinecarboxylic acid; glycine oxazolecarboxylic acid; glycine thiazolecarboxylic acid; L-serine thiazolecarboxylic acid; L-phenyalanine thiazolecarboxylic acid; L-cysteine thiazolecarboxylic acid; L-lysine thiazolecarboxylic acid; O-(phospho-5'-DNA)-L-serine; keratan sulfate D-glucuronyl-D-galactosyl-D-galactosyl-D-xylosyl-L-threonine; L-selenocysteinyl molybdopterin guanine dinucleotide; O4'-(phospho-5'-RNA)-L-tyrosine; 3-(3'-L-histidyl)-L-tyrosine; L-methionine sulfone; dipyrrolylmethanemethyl-L-cysteine; S-(2-aminovinyl)-3-methyl-D-cysteine; O4'-(phospho-5'-DNA)-L-tyrosine O-(phospho-5'-DNA)-L-threonine; O4'-(phospho-5'-uridine)-L-tyrosine; N-(L-glutamyl)-L-tyrosine; S-phycobiliviolin-L-cysteine; phycoerythrobilin-bis-L-cysteine; phycourobilin-bis-L-cysteine; N-L-glutamyl-poly-L-glutamic acid; L-cysteine sulfinic acid; L-3',4',5'-trihydroxyphenylalanine; O-(sn-1-glycerophosphoryl)-L-serine; 1-thioglycine; heme P460-bis-L-cysteine-L-tyrosine; O-(phospho-5'-adenosine)-L-threonine; tris-L-cysteinyl-L-cysteine persulfido-bis-L-glutamato-L-histidino tetrairon disulfide trioxide; L-cysteine persulfide; 3'-(1'-L-histidyl)-L-tyrosine; heme P460-bis-L-cysteine-L-lysine; 5-methyl-L-arginine; 2-methyl-L-glutamine; N-pyruvic acid 2-iminyl-L-cysteine; N-pyruvic acid 2-iminyl-L-valine; heme-L-histidine; S-selenyl-L-cysteine; N6-methyl-N6-poly(N-methyl-propylamine)-L-lysine; hemediol-L-aspartyl ester-L-glutamyl ester; hemediol-L-aspartyl ester-L-glutamyl ester-L-methionine sulfonium; L-cysteinyl molybdopterin guanine dinucleotide; trans-2,3-cis-3,4-dihydroxy-L-proline; pyrroloquinoline quinone; tris-L-cysteinyl-L-N1'-histidino tetrairon tetrasulfide; tris-L-cysteinyl-L-N3'-histidino tetrairon tetrasulfide; tris-L-cysteinyl-L-aspartato tetrairon tetrasulfide; N6-pyruvic acid 2-iminyl-L-lysine; tris-L-cysteinyl-L-serinyl tetrairon tetrasulfide; bis-L-cysteinyl-L-N3'-histidino-L-serinyl tetrairon tetrasulfide; O-octanoyl-L-serine. One of ordinary skill in the art would readily recognize that other post-translational modifications occur.

One of ordinary skill can readily recognize that the methods described herein may be used to detect a variety of post-translational modifications relevant to basic research or to the clinical diagnosis of disease. Examples of the types include, but are not limited to, alkylation, see *e.g*. Saragoni et al., Neurochem. Res. 2000; 25:59-70; Fanapour et. al, WMJ 1999, 98:51-4; Raju et. al, Exp. Cell Res. 1997, 235:145-54; Zhao et al, Mol. Biol. Cell. 2000, 11:721-34; or Seabra, J. Biol. Chem. 1996, 271:14398-404.

Examples of phosphorylation include, but are not limited to, Vanmechelen et al, Neurosci. Lett. 2000, 285:49-52; Lutz et al, Pancreas 1994, 9:418-24; Gitlits et. al J. Investig. Med. 2000, 48:172-82; or Quin and McGuckin, Int. J. Cancer. 2000, 87:499-506.

An example of sulfation includes, but is not limited to, Manzella et. al J. Biol. Chem. 1995, 270S:21665-71.

Examples of post-translational modification by oxidation or reduction include, but are not limited to, Magsino et. al, Metabolism 2000, 49:799-803; or Stief et. al, Thromb. Res. 2000, 97:473-80.

Examples of ADP-ribosylation include, but are not limited to, Galluzzo et. al, Eur. J. Immunol. 1995, 25:2932-9; or Thraves et. al, Med. 1986, 50:961-72.

An example of hydroxylation includes, but is not limited to, Brinckmann et. al, J. Invest. Dermatol. 1999, 113:617-21.

Examples of glycosylation include, but are not limited to, Johnson et. al, Br. J. Cancer 1999, 81:1188-95; Fulop et. al, Biochem. 1996, J. 319,935-40; Dow et. al, Exp. Neurol. 1994, 28:233-8; Kelly et. al, J. Biol. Chem. 1993, 268:10416-24; Goss et. a/, Clin. Cancer Res. 1995, 1:935-44; or Sleat et. al, Biochem. J. 1998, 334:547-51.

An example of glucosylphosphatidylinositide addition includes, but is not limited to, Poncet et. al, Acta Neuropathol. 1996, 91:400-8.

An example of ubiquitination includes, but is not limited to, Chu et. al, Mod. Pathol, 2000, 13:420-6.

An example of a translocation leading to a disease state includes, but is not limited to, Reddy et. al, Trends Neurosci. 1999, 22:248-55.

### The Synthesis of Peptide Compounds

Synthesis and purification of these peptides can be achieved using commercially available peptide synthesisers (*e.g*., Applied Biosystems, Framingham, MA) and protection residues, as described below.

The coupling of the amino acids may be accomplished by techniques familiar to those in the art and provided, for example, in Stewart and Young, 1984, Solid Phase Synthesis, Second Edition, Pierce Chemical Co., Rockford, IL. Amino acids used for peptide synthesis may be standard Boc (Nalpha-amino protected Nalpha-t-butyloxycarbonyl) amino acid resin with the standard deprotecting, neutralization, coupling and wash protocols of the original solid phase procedure of Merrifield (J. Am. Chem. Soc. 1960, 85:2149-2154), or preferably the base-labile N-alphao-amino protected 9-fluorenylmethoxy- carbonyl (Fmoc) amino acids first described by Carpino and Han (1972, J. Org. Chem. 37:3403-3409). Both Fmoc and Boc alpha-amino protected amino acids can be obtained from Fluka, Bachem, Advanced Chemtech, Sigma, Cambridge Research Biochemical, Bachem, or Peninsula Labs or other chemical companies familiar to those who practice this art. In addition, the method of the invention can be used with other N-alpha□-protecting groups that are familiar to those skilled in this art. Many methods of activation may be used in the practice of the invention and include, for example, preformed symmetrical anhydrides (PSA), preformed mixed anhydride (PMA), acid chlorides, active esters, and *in situ* activation of the carboxylic acid, as set forth in Fields and Noble, Int. J. Pept. Protein Res. 1990, 35:161-214. Solid phase peptide synthesis may be accomplished by techniques familiar to those in the art and provided, for example, in Stewart and Young, Solid Phase Synthesis, Second Edition, 1984, Pierce Chemical Co., Rockford, IL; Fields and Noble, Int. J. Pept. Protein Res. 1990, 35:161-214, or using automated synthesizers, such as sold by Applied Biosystems (Framingham, MA).

The following non-classical amino acids may be incorporated in peptides to introduce particular conformational motifs: 1,2,3,4-tetrahydroisoquinoline-3-carboxylate (Kazmierski et al., 1991, J. Am. Chem. Soc. 1991,113:2275-2283); (2S,3S)-methylphenylalanine, (2S,3R)-methyl-phenylalanine, (2R,3S)-methyl-phenylalanine and (2R,3R)-methyl-phenylalanine (Kazmierski and Hruby, Tetrahedron Lett. 1991,); 2-aminotetrahydronaphthalene-2-carboxylic acid (Landis, 1989, Ph.D. Thesis, University of Arizona); hydroxy-1,2,3,4-tetrahydroisoquinoline-3-carboxylate (Miyake et al., J. Takeda Res. Labs. 1989, 43:53-76); beta-carboline (D and L) (Kazmierski, 1988, Ph.D. Thesis, University of Arizona); HIC (histidine isoquinoline carboxylic acid) (Zechel et al., Int. J. Pep. Protein Res. 1991, 43); and HIC (histidine cyclic urea) (Dharanipragada).

The following amino acid analogs and peptidomimetics may be incorporated to induce or favor specific secondary structures: LL-Acp (LL-3-amino-2-propenidone-6-carboxylic acid), a beta-turn inducing dipeptide analog (Kemp et al., J. Org. Chem. 1985, 50:5834-5838); beta-sheet inducing analogs (Kemp et al., Tetrahedron Lett. 1988, 29:5081-5082); alpha-turn inducing analogs (Kemp et al., Tetrahedron Lett. 1988, 29:5057-5060); µ-helix inducing analogs (Kemp et al., 1988, Tetrahedron Lett. 29:4935-4938) and analogs provided by the following references: Kemp et al., J. Org. Chem. 1989, 54:109:115; Nagai and Sato, Tetrahedron Lett. 1985, 26:647-650; DiMalo et al., J. Chem. Soc. Perkin Trans. 1989, p. 1687; also a Gly-Ala turn analog (Kahn et al., Tetrahedron Lett. 1989, 30:2317); amide bond isostere (Jones et al., Tetrahedron Lett. 1988, 29:3853-3856); tretrazol (Zabrocki et al., J. Am. Chem. Soc. 1988, 110:5875-5880); DTC (Samanen et al., Int. J. Protein Pep. Res. 1990, 35:501:509); and analogs taught in Olson et al., J. Am. Chem. Sci. 1990, 112:323-333 and Garvey et al., J. Org. Chem. 1990, 56:436.

### Preparation of Capture Agents

As used herein an agent that "recognizes" a peptide compound or a sequence thereof refers to the ability of this agent to specifically interact with this peptide compound. Capture agents may be generated de *novo* against peptide compounds or selected from libraries of agents. Examples of capture agents include antibodies (*e.g*., polyclonals from animals, monoclonals from hybridomas), single chain antibodies from phage display libraries (Vaughan et al., Nat Biotechnol. 1996, 14:309-14), small peptides (Norman et al. Science 1999, 285:591-5) or RNA-protein fusion libraries (Kreider et al. Med Res Rev 2000, 20:212-5), DNA and RNA aptamers (Kusser et al. J Biotechnol. 2000, Mar; 74:27-38.), small molecules (Macbeath et al. J. Am. Chem. Soc. 1999, 121:7967-7968), random length peptides and proteins (Walter et al. Curr Opin Microbiol. 2000, 3:298-302), as well as natural or recombinant receptor proteins, and their fragments (Alexander and Peters, 2000, Trends in Pharmacological Sciences, Published by Current Trends, London).

In one embodiment the capture agents that bind to the target peptide fragment are themselves peptides and allow peptide-peptide interactions to be characterized.

In a further preferred embodiment the agents that bind to the peptide compounds are antibodies.

In another embodiment only those target peptide fragments known to be produced from a protein of interest by prior knowledge of, for example without limitation, tandem fragmentation analysis (MS/MS) by mass spectrometry are used to produced peptide compounds or capture agents.

### Antibodies

Polyclonal antibodies that may be used in the methods of the invention are heterogeneous populations of antibody molecules derived from the sera of immunized animals. Unfractionated immune serum can also be used. Various procedures known in the art may be used for the production of polyclonal antibodies to a selected peptide compound. In a particular embodiment, rabbit polyclonal antibodies to an epitope of said peptide compound can be obtained. For example, for the production of polyclonal or monoclonal antibodies, various host animals, including but not limited to rabbits; mice, rats, etc, can be immunized by injection with the native or a synthetic (*e.g*., recombinant) version of these peptide compounds.

For preparation of monoclonal antibodies (mAbs) directed toward a selected peptide compound, any technique that provides for the production of antibody molecules by continuous cell lines in culture may be used. For example, the hybridoma technique originally developed by Kohler and Milstein (Nature 1975, 256:495-497), as well as the trioma technique, the human B-cell hybridoma technique (Kozbor et al., Immunology Today 1983, 4:72), and the EBV-hybridoma technique to produce human monoclonal antibodies (Cole et al., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs of the invention may be cultivated in vitro or in vivo. In an additional embodiment of the invention, monoclonal antibodies can be produced in germ-free animals utilizing known technology.

The monoclonal antibodies include but are not limited to human monoclonal antibodies and chimeric monoclonal antibodies (*e.g*., human-mouse chimeras). A chimeric antibody is a molecule in which different portions are derived from different animal species, such as those having a human immunoglobulin constant region and a variable region derived from a murine mAb (see, *e.g*., U.S. Patent Nos. 4,816,567 and 4,816,397).

Antibodies can also be generated using various phage display methods known in the art. In phage display methods, functional antibody domains are displayed on the surface of phage particles that carry the polynucleotide sequences encoding them. In a particular embodiment, such phage can be utilized to display antigen binding domains expressed from a repertoire or combinatorial antibody library (*e.g*., human or murine). Phages expressing an antigen binding domain that binds the antigen of interest can be selected or identified with antigenic peptide compound s, *e.g*., using labeled peptides or peptides bound or captured to a solid surface or bead. Phage used in these methods are typically filamentous phages including fd and M13 binding domains expressed from phage with Fab, Fv or disulfide stabilized Fv antibody domains recombinantly fused to either the phage gene III or gene VIII protein. Phage display methods that can be used to make the antibodies of the present invention include those disclosed in Brinkman et al., J. Immunol. Methods 1995, 182:41-50; Ames et al., J. Immunol. Methods 1995, 184:177-186; Kettleborough et al., Eur. J. Immunol. 1994, 24:952-958; Persic et al., Gene 1997, 187:9-18; Burton et al., Advances in Immunology 1994, 57:191-280; PCT Publications WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, *e.g*., as described in detail below. For example, techniques to produce recombinant Fab, Fab' and F(ab')2 fragments can also be employed using methods known in the art such as those disclosed in PCT Publication WO 92/22324; Mullinax et al., BioTechniques 1992, 12:864-869; and Sawai et al., AJRI 1995, 34:26-34; and Better et al., Science 1998, 240:1041-1043.

Examples of techniques which can be used to produce single-chain Fvs and antibodies include those described in U.S. Patents 4,946,778 and 5,258,498; Huston et al., Methods in Enzymology 1991, 203:46-88; Shu et al., Proc. Natl. Acad. Sci. USA 1993, 90:7995-7999; and Skerra et al., Science 1988, 240:1038-1040.

### Selection of Capture Agents

To select affinity capture agents from libraries of potential capture agents, the peptide compounds may be immobilized, incubated with potential capture agents and washed. The high affinity binders are selected according to any method well-known by one skilled in the art (Vaughan et. al 1996, Nat Biotechnol. 1996, 14:309-14). In one embodiment, peptide compounds can be immobilized on activated hydrogel 1000 x 200µm2 (20µm depth) pads, separated in two dimensions each, each having a different peptide immobilized on it (*e.g*., Vasiliskov et. al. Biotechniques 1999, 27:592-4, 596-8, 600). Libraries of potential affinity capture agents can be incubated with the immobilized peptide compounds in order to select capture agents for each peptide compound. The capture agents can be eluted individually from the hydrogel pads and amplified by, *e.g*., phage propagation (Vaughan et. al., Med Res Rev 2000, 20:212-5). Subsequent rounds of enrichment can be performed on these amplified capture agents by further incubation with similar immobilized peptides. Selection of single high affinity capture agents can be achieved by growing individual phages or cloning of RT-PCR products, for example.

Alternately, when capture agents are antibodies displayed by phage libraries, these phages may be directly selected against peptide compounds.

In a specific embodiment, phage cDNA is ligated with PCR-amplified mRNA light-chain joined to heavy-chain variable regions, isolated from naive splenocytes or bone-marrow stem cells. *E.coli* bacteria are co-transfected with Phage (pilus depolymerization), and with helper phage M13 to allow DNA packaging of the phagemid into phage particles. ScFv (single chain variable fragment) Ab (antibody) is displayed on gpIII, that is, the coat protein used to bind to and infect *E.coli.*

For phage selection, one can use a 96 well plate containing multiple 25-mers to specific peptides (5 per different peptide). The non-binders are washed away in each well. The binders then are used to re-transfect *E.coli* bacteria and grown to confluency. Phages are harvested, and re-exposed to the equivalent peptide to weed out non-specific binders (*e.g*., the ones that bind to plastic or any inherently sticky phagemids).

After three rounds of selection, *E. coli* samples that contain phagemids are spread onto plates containing a selection factor (1/10, 1/100). Resulting colonies are chosen and grown up in suspension containing the selection factor. The phages secreted into the medium are then recovered.

Phages that display antibodies are used in ELISAs to identify strong binders that can be tracked back to the original clones.

### Arraying and Immobilization of Capture Agents

The capture agents selected according to the method described above are immobilized on a solid support to form an array. Selection of the solid support and the methods for immobilization depend on the type of capture agents (proteins, nucleic acids, etc.). Immobilization may be performed through covalent or non-covalent binding. In a preferred embodiment, the capture agents are covalently bound to the surface of the solid support via reactive groups or cross-linking agents.

When capture agents are proteins, such as receptors or antibodies, covalent immobilization may be achieved through free cysteines using thiol reactive surfaces, or through lysines using amine reactive surfaces.

Alternatively, heterobifunctional crosslinkers may be used to cross link- NH groups the surface to sulfhydryl (SH) groups or cysteine residues. BMPS (beta-maleimidopropionic acid N-hydroxysuccinimide ester, *e.g*. purchased from SIGMA) can be used to cross link -NH groups to sulfhydryl (SH) groups on cysteine residues of antibodies may be used for that purpose.

Non-covalent interaction may be achieved by using Avidin- or Streptavidin-coated surfaces and biotinylated affinity capture agents; protein-A or protein-G coated surfaces and Fc containing affinity capture agents (*e.g*., immunoglobulins, etc.); and metal-chelate surfaces and Histidine tagged affinity capture agents.

In one preferred method, capture agents are multiple single chain antibodies (SCAs) specific to different peptides. They are purified from phages and solubilised in PBS at similar concentrations (about 0.1 mg ml-1). The specific SCAs are placed in multiwell plates (*e.g*., 96 well). These plates can then be used in a microarray system. It is preferable when dispensing proteins to employ a system that does not use steel pins or contact dispensing equipment. Thus, a preferred system is the Packard BCA Piezo robot (U.S. Patent No. 5,927,547), which dispenses small volume drops (less than 1nL) from a glass capillary from above the surface of the microarray substrate. A preferred substrate is aldehyde activated polyacrylamide pads immobilized on glass or oxidised silicon. A preferred size for the polyacrylamide pads is 2cm x 2cm x 20µm. about 300pL of each SCA is dispensed into a discrete area within the polyacrylamide pad to create a 2-dimensional array in a 3-dimensional structure. The resulting spot is about 200µm in diameter. The free aldehydes within the polyacrylamide react with the amines in the SCAs so that the SCAs are covalently immobilized. Once this reaction is complete (30 minutes after dispensing) any remaining aldehydes are reduced with sodium borohydride or blocked using reagents containing amino groups (*e.g*., TRIS buffer) and/or free amino acids.

Another preferred substrate is hydrogel on which capture agents such as antibodies may be immobilized, *e.g*., via -NH group of Lysines to form a Schiff base with aldehyde groups present in the hydrogel. Lysines also provide free -NH groups that can be cross-linked to -SH groups of cysteine residues in antibodies (*e.g*., via BMPS).

Hydrogel immobilization has given better results in terms of signal to noise ratio and fluorescence specifically when compared to glass or silicon substrates.

In another preferred method the substrate is produced by casting a thin agarose gel on a glass slide, using suitable gaskets or other spacers, and siliconised cover glass (or cover slips or similar solid surface for producing uniform thickness gel). Following casting, the gel is activated with CNBr. The use of agarose gels is preferred over commercial Hydrogels (e.g. from Packard Instrument Co., Meriden, CT) as agarose retains moisture more efficiently and thus allows for spotting of dehydration-sensitive reagents. Another advantage of using thin agarose gel films is that they allow for faster diffusion of compounds due to a larger pore size. Therefore, shorter washing steps and lower affinity capture agents may be used. This is in contrast to acrylamide based Hydrogels, which require long washing times and, therefore, higher affinity capture agents. Further, agarose-based supports can be dehydrated (dried out) following incubation/washing steps, prior to scanning to improve signal and resolution. A further advantage of 3-dimensional gel-based immobilization techniques is that with careful choice of physical and physicochemical gel properties it is possible to allow target peptide fragment-capture agent binding to most closely approximate the binding likely to occur in free solution ie. less impeded by solid surfaces.

In yet another embodiment, single chain Fv antibodies are histidine-tagged for the previously-described isolation step. The histidine tag can further be used for immobilization, *e.g*. using metal chelate affinity on nickel-modified surfaces, for instance.

Furthermore, this system allows multiple affinity capture agents, specific to different target peptide fragments from the same or different proteins, to be put in the same spot, particularly when MALDI-MS is being used as a detection method. Additional immobilization chemistries are possible using other affinity agents and array substrates (*e.g*. Lin, Science 1997, 278:840-843).

Other examples of immobilization include using thin membranes (such as nitrocellulose, nylon (charged or uncharged), PVDF and/or their derivatives) attached to or immobilized on glass slides or other solid surfaces. For example attaching commercially available membranes (nitrocellulose, supported nitrocellulose, nylon, charged nylon or PVDF membranes from Schleicher & Schuel, UK Ltd., Amersham, Millipore and other suppliers) to glass slides or by using CAST™ (layer of Nytran® SuPerCharge positively charged nylon membrane affixed to the glass) and FAST™ slides (microporous polymeric surface cast onto glass) from Schleicher & Schuel, UK, Ltd. may lower costs significantly. For this method, affinity capture agents may be dispensed using piezo robots similar to the one described above for SCAs. One of the advantages of using nitrocellulose or nylon based supports is that they have a much higher protein binding capacity compared to Hydrogels or 2D solid supports (glass, silicon, plastic etc.). A higher binding capacity results in a stronger fluorescent signal, and therefore allows for the use of binders having lower affinity. Various solid support materials that may be used in the microarray are as follows:
- Binding capacity (highest to lowest):
   a) immobilized nitrocellulose ~ immobilized nylon (charged > uncharged)
   b) FAST™ ~ CAST™
   c) Hydrogel
   d) derivatized solid surfaces (glass; silicon, plastics)
- Spot geometry (best to worst):
   a) derivatized solid surfaces (glass, silicon, plastics)
   b) FAST™ ~ CAST™
   c) immobilized nitrocellulose ~ immobilized nylons
- Variability in spotting (least variable to most variable):
   a) FAST™
   b) CAST™
   c) immobilized nitrocellulose
   d) immobilized nylons
   e) Hydrogels
- Moisture retaining (best to worst):
   a) agarose pads
   b) acrylamide pads (Hydrogels)
   c) immobilized membranes or derivatized solid surfaces (glass, silicon, plastics)
- Tolerance to glycerol in the spotting mixtures (highest to lowest):
   a) FAST™ ~immobilized nitrocellulose
   b) CAST™ ~ immobilized nylons

   a) Hydrogel

Another preferred immobilization technique uses a combination of non-covalent affinity immobilization coupled to covalent cross-linking. This technique is especially advantageous for immobilizing affinity capture agents such as antibodies, as it allows the antibodies to be attached in an orientation whereby their active sites are easily accessible, and at the same time, remain covalently cross-linked to the solid support: This technique includes, but is not limited to, using Hydrogels (Packard Instrument Co., Meriden, CT) for immobilization of protein-A or protein-G, followed by treatment with glutaraldehyde (high concentration for short time): this results in covalent cross-linking of the protein-A/G to the solid support, and also in aldehyde derivation of solution-exposed amino groups of the protein-A/G. Substrates are then washed to remove the excess free glutaraldehyde, while bound aldehyde groups remain un-blocked. Subsequently, Fc containing antibodies (e.g. IgG) are spotted or dispensed onto the derivatized protein-A/G, which then binds the Fc fragments of the antibodies. Following incubation, protein-A/G eventually cross-links with the bound antibodies through aldehyde-derivatized groups on the protein-A/G and available amino groups on the Fc fragments. This approach results in a high density antibody immobilization, with the antibody being correctly oriented and covalently cross-linked to the solid support. Glutaraldehyde pre-treatment of the immobilized Protein-A/G prior to antibody spotting is a preferred technique because it avoids incubating the affinity capture agents (i.e., the antibodies) with the cross-linking agent (i.e. glutaraldehyde), which could result in the derivatization of amino groups of the antibodies and, therefore, loss of the high affinity binding sites.

It is well known that many proteins contain nucleic acid-binding domains. As such the use of nucleic acids arrays capture agents will allow the capture of proteins containing nucleic acid-binding domains. As used herein, the term "nucleic acid array" refers to "gene chips" and related arrays of oligonucleotides, cDNAs, and other nucleic acids, which are well known in the art (see for example the following: U.S. Patent Nos. 6,045,996; 6,040,138; 6,027,880; 6,020,135; 5,968,740; 5,959,098; 5,945,334; 5,885,837; 5,874,219; 5,861,242; 5,843,655; 5,837,832; 5,677,195 and 5,593,839). In the context of the present invention, the nucleic acids that are attached to the solid support are preferably DNA and RNA aptamers.

The nucleic acid is attached to a solid support, which may be made from glass, silicon, plastic (*e.g*., polypropylene, nylon), polyacrylamide, nitrocellulose, or other materials. A preferred method for attaching the nucleic acids to a surface is by printing on glass plates, as is described generally by Schena et al., Science 1995, 270:467-470. This method is especially useful for preparing microarrays of cDNA. See also DeRisi et al., Nature Genetics 1996, 14:457-460; Shalon et al., Genome Res, 1996, 6:639-645; and Schena et al., Proc. Natl. Acad. Sci. USA 1995, 93:10539-11286.

A preferred method of making microarrays is by use of an inkjet printing process to bind genes or oligonucleotides directly on a solid phase, as described, *e.g*., in U.S. Patent No. 5,965,352.

Other methods for making microarrays, *e.g*., by masking (Maskos and Southern, Nucl. Acids Res. 1992, 20:1679-1684), may also be used. In principal, any type of array, for example, dot blots on a nylon membrane (see Sambrook et al., Molecular Cloning A Laboratory Manual (2nd Ed.), Vol. 1-3, Cold Spring Harbor Laboratory, Cold Spring Harbor, N.Y., 1989, could be used, although, as is recognized by those of skill in the art, very small arrays are preferred because sample volumes are smaller.

### Reparation of the Biological Sample:

The present invention contemplates determining the presence of a protein in a sample. Any sample that is likely to contain a protein of interest may be tested. Such samples, referred to herein as biological samples, include body fluid (*e.g*. blood, serum, plasma, saliva, urine, plural effusions or cerebrospinal fluid), a tissue sample (*e.g*., a biopsy, blood cells, smears) or homogenates and extracts, including cytoplasm, membranes, and organelles thereof..Cell cultures and culture fluid are also biological sample.

The sample may be pre-treated to obtain a protein preparation substantially free of contaminants. Such a treatment may comprise fractionation, differential extraction (membrane and cytosolic fractions); selective depletion (*e.g*., for removal of albumin, haptoglobin, immunoglobin G); and application to any specific affinity column (*e.g*., Mannose-6-Phosphate receptor for lysosomal enzymes; Sleat and Lobel, J Biol Chem 1997, 272:731-8).

Proteins present in the sample may be in native form or denatured (Wilkins MR; et al.,1996, Biotechnology (N Y) 14(1):61-5, *e.g*. by dissolving in 6M guanidine HCl (or 6-8M urea), 50 mM Tris-HCl (pH8), 2-5 mM DTT (or 2-mercaptoethanol). Proteins present in the sample may also be pre-treated with eg. glycosidases (glycoprotein deglycosylation kit available from CN biosciences, UK. Ltd.) to remove glycosylation side chains or other means of predictably varying post-translational modifications. The sample is then subjected to conditions that allow enzymatic or chemical cleavage of the individual proteins into target peptides. Preferably, this method uses the same conditions initially used to digest or predict digestion of the protein sequence selected for generation of target peptide fragments from which the capture agents were prepared. However other cleavage agents may be used on the condition that they do not disturb the capture agent binding site, *e.g*., antigenic determinant of the target peptide fragment

To break disulfide bonds, which link proteins by cysteine residues, and to prevent residues from recombining, a reduction/alkylation step can be performed prior to proteolysis. Dithiothreitol (DTT) may be used for reduction and iodoacetamide may be used for carboxyamidomethylation of cysteine.

Reproducible peptide fragments can be generated from protein preparations using proteolytic and chemical methods (*e.g*. Schevchenko et al. Analytical Chemistry 1996, 68:850-858; Houthaeve et al., FEBS Letters 1995, 376:91-94; Wilkins *et* al., 1997, Springer ISBN 3-540-62753-7). The cleavage may be enzymatic cleavage. Preferably, it is a selective enzymatic cleavage, *e.g*. with arginine endopeptidase (ArgC); asparatic acid endopeptidase N (aspN); chymotrypsin; glutamic acid endopeptidase C (gluC); lysine endopeptidase C (lysC); trypsin; or V8 endopeptidase.

Trypsin is preferred, as it specifically cleaves proteins at the C-terminus of Arg or Lys. For trypsinolysis, mammalian cells *e.g*. Jurkat cells, can be harvested and lysed in RIPA buffer (20 mM Tris-HCl, pH 7.4, 150 mM NaCl, 1% Triton X-100 (v/v), 1% deoxycholate (w/v), 0.1% sodium dodecyl sulphate (w/v)), followed by addition of high purity porcine-trypsin at a concentration of 10 ng/ml, and incubated for 16 hours at 37° C. The trypsin can be neutralized by the addition of a cocktail containing serine protease inhibitors. Tryptic peptides then can be purified and buffered with PBST by gel filtration. Alternatively, trypsin can be removed by immuno-precipitation or inactivated by boiling.

The tables below show examples of cleavage agents and cleavage sites.

**Table 2: Protein cleavage using Proteolytic Enzymes**

| Enzymes | Preferred cleavage site |
|---|---|
| Ancrod | Arg-X, Arg-Gly |
| Bromelain | C-terminal to Lys, Ala and Tyr |
| Chymotrypsin | C-terminal to hydrophobic residues, e.g., Phe, Tyr, Trp. Less sensitive with Leu, Met, Ala |
| Clostripain | C-terminal to Arg residues |
| Collagenase | N-terminal to Gly (X-Gly) in Pro-X-Gly-Pro |
| Elastase | C-terminal to amino acids with small hydrophobic side chains |
| Endoproteinase Arg-C | C-terminal to Arg residues |
| Endoproteinase Asp-N | N-terminal to Asp and Cys |
| Endoproteinase Glu-C | C-terminal to Asp and Glu |
| Endoproteinase Lys-C | C-terminal to Lys |
| Factor Xa | C-terminal to Arg in Gly-Arg-X |
| Ficin | uncharged or aromatic amino acids |
| Follipsin | Arg-X |
| Kallikrein | C-terminal to Arg in (Phe-Arg-X or Leu-Arg-X) _{.} |
| Pepsin | Broad specificity; preference for cleavage C-terminal to Phe, Leu, and Glu |
| Thermolysin | N-terminal to amino acids with bulky hydrophobic side chains, e.g., Ile, Leu, Val, and Phe |
| Thrombin | C-terminal to Arg |
| Trypsin | C-terminal to Lys and Arg |
| V8 protease | C-terminal to Glu, less active with Asp |

**Table 3: Chemical cleavage of proteins**

| Chemistry | Cleavage site |
|---|---|
| Cyanogen bromide | Trp, (Met) |
| Formic acid | Asp - Pro |
| HCl | Asp-X, X-Asp |
| Hydroxylamine (alkaline pH) | Asn - Gly |
| N-bromosuccinimide (NBS) or N- chlorosuccinimide | Trp |
| 2-Nitro-5-thiocyanobenzoate (NTCB) | Cys |

Regardless of the type of proteolytic agent used, the optimum digestion time to produce the desired quality of peptide fragments may be determined for example by collecting aliquots every 2 hours and after an overnight digest.

Target peptide fragments are preferably peptides that are between 6 and 25 residues in length.

In one embodiment a sample containing proteins of interest can be split into two or more aliquots and each aliquot treated with a different enzyme or chemical agent to produce complementary overlapping target peptide fragments. Each proteolytically cleaved sample is then analysed as described herein.

### Peptide Labeling

Target peptide fragments may be labeled according to any standard method to facilitate detection.

In one embodiment, the target peptide fragment may be directly labeled. In another embodiment, a labeled secondary reagent may be used to detect binding of the target peptide fragment to the solid support containing the capture agent. Binding may be detected by *in situ* formation of a chromophore by an enzyme label. Suitable enzymes include, but are not limited to, alkaline phosphatase and horseradish peroxidase (HRP). Other labels for use in the invention include fluorescein isothiocyanate (FITC), phycoerythrin (PE), Texas red (TR), rhodamine, free or chelated lanthanide series salts, especially Eu³⁺, to name a few fluorophores), chemiluminescent molecules, radio-isotopes (¹²⁵I, ³²P, ³⁵S, chelated Tc, etc.) or magnetic resonance imaging labels. Two color assays may be performed using fluorophores that emit at different wavelengths.

Metabolic (or biosynthetic) labeling with [³⁵S]-methionine, is also contemplated as well as labeling with [¹⁴C]-amino acids and [³H]-amino acids (with the tritium substituted at non-labile positions).

A preferred method makes use of NHS ester Bodipy TMR , Molecular Probes, Leiden, the Netherlands. Other methods include the use of Fluorophores - iodoacetamide, maleimide or succinimide groups (*e.g*., Molecular Probes) Cy3, Cy5 or TAMRA dyes for example via labeling cysteines or lysines (*e.g*., Alexa Fluor dyes).

One can also use activated biotin attached through a maleimide group to cysteine residues. Detection is performed using streptavidin- HRP to amplify signal. Reactive residues may also be included within the selected peptide fragment. For example, cysteines are readily modified by maleimide and iodoacetamide containing reagents. This allows enrichment for those peptides from the cleaved pool via *e.g*., iodoacetamide-biotin (Molecular Probes, Leiden, Netherlands) and streptavidin capture. Further fluorescent signals can be increased via iodoacetamide-Bodipy TMR (Molecular Probes, Leiden, Netherlands), for example.

### Contacting of Target Peptide Fragments with Immobilized Capture Agents

Labeled or unlabeled target peptide fragments (about 1 mg ml⁻¹) in PBST or another suitable buffer are incubated with the array of immobilized capture agents. An example of incubation Buffer is 1 x PBS, pH 7.4, phosphate buffer (0.01 M) plus NaCl (0.137M), KCI (0.0027M), without Tween detergent.

The incubation may proceed at room temperature or at 37°C in a sealed humidified chamber from 1 hour to 24 hours, in a volume of about 25µl under a coverslip or 65µl in a chamber formed with gaskets (Clontech). No agitation is necessary.

Target peptide fragments that have bound non-specifically to the array are removed by 2 x 15 minute washes in PBST (200ml) at room temperature followed by 3 rinses in distilled water. The water rinses remove salt and detergent, which can interfere with detection methods.

### Detection and Quantitation of Captured Target Peptide Fragments

Any method known in the art for detecting the binding of the labeled target peptide fragments to the capture agents may be performed according to the present invention. Where target peptide fragments are known and the affinity of the capture agent for each target peptide fragment can be measured by techniques known in the art, any array can be calibrated with respect to each target peptide. When one or more target peptide fragment-capture agent pairs of known specificity and known affinity are available for use in an assay accurate semi-quantitative or quantitative results can be obtained with the array.

### Fluorescence Analysis

When fluorescent labeling is used, the fluorescent signal may be analyzed by various methods, including fluorescence intensity or polarization (Onnerfjord et al., J. Immuno. Methods 1998, 213:31-9), fluorescence correlation spectroscopy (Tetin et al., Methods 2000, 20: 341-61), phase sensitive fluorescence, fluorescence anisotropy, FRET (Fluorescence Resonance Energy Transfer), BRET (Bioluminescence Resonance Energy Transfer), or time resolved fluorescence. Alternatively, radioisotope labeling (Top Count NXT Microplate Scintillation and Luminescence Counters from Packard), or surface plasmon resonance (SPR-Regnault et al. Br. J. Haematol 2000, 109:187-94) may also be contemplated.

In a preferred embodiment, microarrays are washed and placed in commercially available confocal laser scanners, *e.g*., ScanArray 3000 (Gsi lumonics, USA). The amount of fluorescence per spot (capture agent) is relative to the amount of captured labelled peptide which, in turn, is relative to the amount of protein containing that peptide in the protein preparation. Software such as Optiquant can be used to accurately measure the fluorescence from each spot.

Preferably fluorescence assays are performed on hydrogel or agarose gel arrays. An example of arrays for fluorescence assays is 12x12mm hydrogels (20µm thick) on glass microscope slides. These dimensions could be increased for higher numbers of antibodies to be immobilized.

### Mass Spectrometry

The captured target peptide fragments can be analyzed by mass spectrometry; this can be using primary mass spectrometry (MALDI-TOF MS) or tandem mass (fragmentation) spectrometry (MS/MS); both of these are discussed in some detail below.

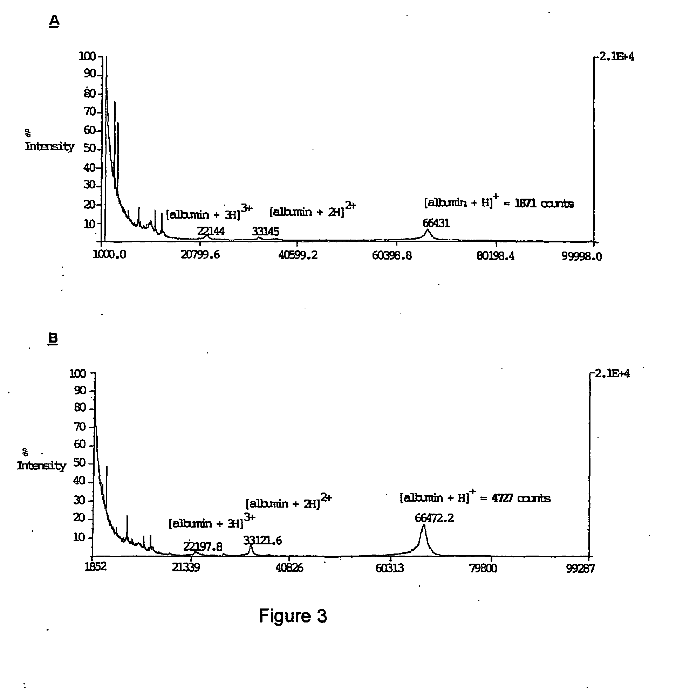

In a preferred embodiment of this invention, the peptides are initially analyse using matrix-assisted laser-desorption time-of-flight mass spectrometry (MS). This instrument configuration is used to determine accurately the molecular weights (less than 100 parts-per-million (ppm)) of the modified and unmodified peptides. It is generally accepted that MALDI MS is a non-quantitative method. However, this is usually due to variabilities in sample preparation where large volumes and non-homogenous matrix crystals are produced. Preferably MALDI assays are performed on silicon arrays. An example of an array for MALDI is 200µm circular gel pads at 350µm centres, on oxidised silicon. A hydrophobic surface (repellent surface) between gelpads further provides a more focused matrix/protein spot for MALDI, thereby improving signal upon quantitation. The spots produced using the system of this invention (Packard BCA Piezo robot (U.S. Patent No. 5,927,547), Packard Instrument Co., Meriden, CT) system are less than 200□µm in diameter. The same system can deliver about 300pL of MALDI matrix (e.g. DHB, sinapinic acid) to the exact position of the affinity capture agent-target peptide fragment spot to create a homogeneous target peptide fragment/matrix crystal. Desorption/Ionization (Karas, et al. Ion Processes, 1987, 78, 53-68 or Zenobi, et al. Mass Spectrom. Rev. 1998, 17, 337-366) from this crystal in a MALDI-MS (*e.g.* Perseptive Voyager) yields a mass spectrum where the height of the peak is relative to the amount of captured target peptide fragment (labeled or unlabeled) which in turn is relative to the amount of protein containing that target peptide fragment in the spotted sample. This detection system also verifies the identity of the captured target peptide fragments by way of their measured mass. The relative abundance of individual proteins present in ovary and serum samples was analysed by antibody arrays. Protein extracts (1µg each) were incubated with identical arrays of antibodies immobilised on Hydrogel pads. Arrays were washed and analysed by MALDI-TOF-MS. Spectra for the anti-albumin antibody mediated captured of albumin from ovary (A) and serum (B) are shown (Figure 3).

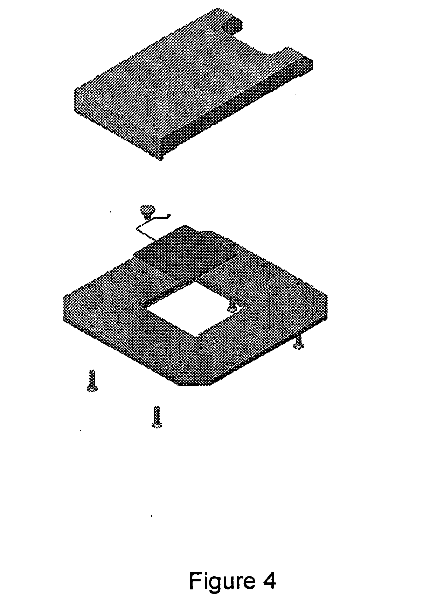

Figure 4 shows a modified target holder for a Perseptive Voyager to hold a silicon-bound polyacrylamide pad.

Following the MALDI-TOF MS analysis the identified masses excluding those ignored are collected and used to search a listing containing sequence information produced by calculating *in silico* fragments which would be obtained by proteolysis of proteins of interest with an appropriate endopeptidase or chemical. The data collected using MALDI-TOF (MS) is represented as a list of parent ion masses. Masses due to the presence of the capture agent can be ignored and analysis focused on masses arising from the target peptide fragments. Intensities of each mass (m/z) feature in the mass spectrum are measured by methods known to those skilled in the art or as specified in the PCT Application number PCT/GB01/04034 filed on 10th September 2001. Where more than one target peptide fragment is available for each protein of interest the intensities of masses in the mass spectra of such fragments can be normalized across such a plurality of signals resulting in greater accuracy and reliability.

Only as necessary, where an identification is not not possible or is ambiguous or when further confimatory data is required, for example for the validation or in development of an array, further analysis of the sample/matrix spot can be performed using any standard method of tandem mass spectrometry (MS/MS) and in particular using MALDI-TOF/TOF (Applied Biosystems, Framingham, MA) or MALDI II Q-TOF (Micromass) or Q-STAR (Sciex) all of which are systems which continue MALDI MS with tandem mass spectrometry. This generates a fragmentation spectrum which is used to generate sequence information.

Database searching of the primary mass data provided by MALDI-TOF MS can be used to identify possible post-translational modifications (PTMs) of peptides. Where there is more than one possible site of post-translational modification tandem mass spectrometry (MS/MS) can be used to provide specific information on the site of such PTMs. For example high energy CID provided by MALDI-TOF/TOF MS has been shown to unambiguously establish the site of peptide phosphorylation (Analysis of Post-Translational Modifications using a MALDI-TOF-TOF Mass Spectrometer, DeGnore *et al.* Poster presentation at the 49th ASMS conference on Mass Spectrometry and Allied Topics, Chicago).

### Apparatus

Various type of apparatus, typically microprocessor (*i*.*e*., computer) controlled, are available for the detection and quantitation of captured peptides fragments, whether by detecting binding of peptide to capture agent, or the absence of label (as detailed below). In particular, fluorescence detection and mass spectrometry employ well-known types of apparatus, *e.g.*, as set forth in the references noted above. The invention further specifically contemplates adapting such apparatus for the specific analysis of microarrays of the invention. In some respects, the robust, standardizable, uniform assays of the present invention (because of binding of peptides to the capture agents) permit adaptation of specific features of the apparatus, including but not limited to incubation time, detection parameters, and processing software, that would be cumbersome if adapted for protein-specific capture agent microarrays.

In particular, an apparatus of the invention, whether for label detection (*e*.*g*., fluorescence) or direct detection of peptide binding (such as mass spectrometry), can be specifically adapted to detect peptide binding in an array of the invention. In particular, such an adaptation may include specific software designed for processing detection data.

In a specific embodiment, software specifically evaluates diagnostic arrays for the presence and amount of key disease markers. The software processes the detected peptides against a database of known markers for particular cellular conditions, and provides as output not raw binding intensity data but a most likely diagnosis. Such an apparatus has clear application in commercial diagnostic laboratories, where the volume of material to be analyzed is high and the capabilities of the technicians are not at the level of Ph.D. scientists.

### Applications

Diagnosis represents a major application of the method of the invention. In one embodiment, a protein or a peptide that is different expressed in a disease can be detected in a biological sample and noted as a marker of the disease or change in biochemical status.

Examples of such markers include Cystatin C for renal dysfunction, (Fliser D. and Ritz E., Am. J. Kidney Dis. 2001, 37(1): 79-83); prostate-specific antigen (PSA) for prostate cancer, (Millenbrand et al., Anticancer Res., 2000, 20(6D): 499-6); Angiotensin II/ACE for heart failure (Kim SD; Biol. Res. Nurs. 2000, 1 (3): 210-26).

### Use of the Method of the Invention in Diagnosis

Data produced by the method of the invention can be analysed by sophisticated statistical techniques including uni-variate and multi-variate ananlysis tools. The following steps can be used to identify target peptide fragments arising from proteins which show an association with a disease or biochemical status.

### 1. uni-variate differential analysis tools

Changes such as fold changes, Wilcoxon rank sum test and t-test, are useful in determining the significance of the expression values of each target peptide fragment and its corresponding protein of interest

### 2. multi-variate differential analysis

The first step is to identify a collection of target peptide fragment signal responses that individually show significant association with any particular condition. The association between the identified proteins and any particular condition need not be as highly significant as is desirable when an individual protein is used in diagnosis. Any of the tests discussed above (fold changes, wilcoxon rank sum test, etc.) can be used at this stage. Once a suitable collection of target peptide fragments has been identified, a sophisticated multi-variate analysis capable of identifying clusters can then be used to estimate the significant multivariate associations with said disease or biochemical status.

Linear Discriminant Analysis (LDA) is one such procedure, which can be used to detect significant association between a cluster of variables and the disease or perturbed biochemical status. In performing LDA, a set of weights is associated with each variable so that the linear combination of weights and the measured values of the variables can identify the disease state by discriminating between subjects having a disease and subjects free from the disease. Enhancements to the LDA allow stepwise inclusion (or removal) of variables to optimize the discriminant power of the model. The result of the LDA is therefore a cluster of target peptide fragments and their corresponding proteins that can be used, without limitation, for diagnosis, prognosis, therapy or drug development. Other enhanced variations of LDA, such as Flexible Discriminant Analysis permit the use of non-linear combinations of variables to discriminate a disease state from a normal state. The results of the discriminant analysis can be verified by post-hoc tests and also by repeating the analysis using alternative techniques, such as classification trees.

A further category of target peptide fragments and their corresponding protein can be identified by qualitative measures by comparing the percentage presence of proteins of interest in one group of samples (e.g., samples from diseased subjects) with the percentage presence of a protein of interest in another group of samples (e.g., samples from control subjects). The "percentage presence" of a protein is the percentage of samples in a group of samples in which the protein of interest is detectable by the detection method of choice. For example, if a protein of interest is detectable in 95 percent of samples from diseased subjects, the percentage feature presence of that the protein of interest in that sample group is 95 percent. If only 5 percent of samples from non-diseased subjects have detectable levels of the same protein of interest, detection of that protein of interest in the sample of a subject would suggest that it is likely that the subject suffers from the disease.

The method of the present invention can assist in monitoring a clinical study, *e.g.*, to evaluate drugs for therapy of a disease. For example, candidate molecules can be tested for their ability to restore levels of protein in a diseased subject to levels found in control subjects or, in a treated subject, to preserve levels of protein at normal values. The levels of one or more proteins of interest can be assayed.

In another embodiment, the method of the present invention is used to screen candidates for a clinical study to identify individuals having a disease; such individuals can then be either excluded from or included in the study or can be placed in a separate cohort for treatment or analysis. Procedures for these screens are well known in the art.

Diagnosis of cancers, such as breast cancer or prostate cancer are of particular interest. Many proteins of interest associated with various diseases or responses have already been identified by the applicants (see below). Reference to such proteins of interest can be found below in International Patent Publications and Applications.

| Disease State | Publication No. | Application No. | Application Date |
|---|---|---|---|
| Breast Cancer | WO 00/55628 WO 01/13117 WO 01/62914 WO 01/63288 WO 01/63289 WO 01/63290 | | |
| Hepatoma | WO 99/41612 WO 01/13118 | | |
| Schizophrenia | WO-01/63293 | | |
| Rheumatoid Arthritis | WO/99/47925 | | |
| Bipolar Affective Disorder | WO 01/63294 | | |
| Unipolar Depression | WO 01/63294 | | |
| Alzheimer'sDisease | | WO 01/75454 | 03-Apr-01 |
| Breast Cancer | | WO 01/71357 | 20-Mar-01 |
| Vascular Dementia | | WO 01/69261 | 14-Mar-01 |

Arrays of the invention may be constructed using proteins and their corresponding target peptide fragments identified in the above applications. Arrays of the invention may also be useful to detect proteins associated with a particular biological pathway, *e.g.*, by detecting cell surface proteins or antigens. These include receptors, such asthose with 7-transmembrane receptordomains/angiotensin II-Receptor, cholecystokinin/gastrin receptor IL-8-Receptor, endothelin receptor, EGF-Receptor, beta-2-adrenergic receptor, etc.or 5-HT receptors, as well as cell adhesion molecules, signal transduction molecules and adaptor molecules. This may also include the capture of proteins associated with particular protein classes such as but not limited to membrane proteins. Arrays could be designed to capture specific domains, proteins containing particular structures, nucleic acid-binding sites and target peptide fragments associated with an intracellular compartment such as, but not limited to the golgi.

Results obtained by analyzing proteins in samples of interest can be stored in a database and referenced subsequently..For example and without limitation, use of arrays of the invention to monitor patients with neurodegenerative disease or cancer allows longitudinal results to be maintained and monitored with time. Each new result can be compared with previous results from the same patient allowing the states of the disease to be monitored.

In one embodiment an array of the invention comprises the array and a computer readable medium to store the analytical output of the array. In a further embodiment the analytical output can be transmitted electronically to a remote computer location and database.

### Competitive Format of Protein Arrays

The concentration of reagents (target peptide fragments and affinity capture agents) in solution, affinity of interaction, and signal strength (amount of bound capture agent-- target peptide fragments pairs) are interrelated parameters. Therefore, for an array experiment in which a number of different capture agent-target peptide fragment pairs are used, the actual affinity of capture molecules may need to be adjusted in accord with the corresponding target peptide fragment concentration in order to be quantitative for every pair of the target peptide fragments. The preferred embodiment of this principle is generating small protein arrays for specialized applications. The preferred format for larger arrays (including generic protein arrays) is a competitive format. The two most preferred embodiments of a competitive format are illustrated below.

In one such embodiment, the affinity capture agents are immobilized in an array format and are contacted with one or more of the target peptide fragments in the sample (for example a mixture of proteins or peptides) in the presence of labeled synthetic peptides corresponding to the set of immobilized affinity capture agents used in the production of the array under conditions that permit binding of peptide fragments to the capture agents. The preferred set of peptides used for labeling should be made from the peptide compounds used for the production of the affinity capture agents, or from the peptides containing sequences used in the production of affinity capture agents.

Another preferred embodiment of the competitive format employs synthetic peptides or peptide compounds that are immobilized on the surface of a solid support. In this embodiment, corresponding labeled affinity capture agents are contacted with the array in the presence of a test sample (comprising of a mixture of peptides obtained after enzymatic or chemical digestion of a test sample). Advantages of the described competitive format include firstly, the possible compensation for variability in the affinities of capture agents and/or variability in the concentration of protein, and hence peptides, in the test samples can be achieved by varying concentration of a displacement reagent (synthetic peptide) or an affinity capture agent. This allows for identical protein arrays to be used against a variety of different test samples. Secondly, the use of labeled synthetic peptides or purified affinity capture agents means there is no requirement for labeling of test sample. This allows more efficient labeling and results in a lower background during binding. The following examples illustrate the invention without limiting its scope.

### Example 1: Preparation of Microarrays

### 1. Generation of polyacrylamide pads

A standard 75mm x 25mm microscope slide is modified by wiping with bind-silane (Sigma-Aldrich) and coverslips of any size are modified with repel-silane (Sigma-Aldrich). 50µl of 8% acrylamide-bisacrylamide (19:1) containing ammonium persulfate (Sigma-Aldrich) and TEMED (Sigma-Aldrich) is spotted onto the modified slide, covered with a modified cover-slip and allowed to polymerise.

### 2. Generation of agarose pads

A warm solution of low melting point agarose (0.5 to 1 % in water) was applied to a standard microscope slide (optionally treated with a bind silane), attached to a GeneFrame gasket (ABgene, Surrey, UK), and covered with a repel silane-treated coverslip. Gels can be used immediately or stored under cover slips at +4°C in sealed containers. Agarose gels are activated using CNBr. Gel-covered slides are equilibrated with 50 mM sodium carbonate buffer, pH 10.5, followed by incubation in 200 mM CNBr/50 mM sodium carbonate, pH10.5. Subsequent washes with large.volumes of 100 mM sodium citrate buffer, pH 6.5, and water are performed. Slides are now ready for attachment of capture agent or can be stored at +4°C in 100 mM sodium citrate/ 0.1 % sodium azide (w/v).

### 3. Affinity agent attachment

Affinity agent attachment can be performed in a variety of ways, which can be categorized into three groups: covalent attachment, non-covalent attachment and a' combination of the covalent and non-covalent attachment.

### a) Example of covalent attachment techniques

Silicon slides with polyacrylamide gel pads (generated as described above or obtained from Packard Bioscience, Meriden, Connecticut, USA) are treated with glutaraldehyde (10-50%) overnight (optionally followed by 2% TFA for 5 min), extensively washed with water, and air-dried. Affinity capture agents (as previously defined) are dispensed using an arrayer (for example BioChip Arrayer with Piezo-tips, Packard Bioscience, Meriden, Connecticut; USA) or by treating the whole or part of the slide with the solution containing the affinity capture agents. Slides are incubated with the affinity capture agent in a sealed, humidified chamber overnight. To block the remaining reactive sites, and to remove unbound antibodies, slides are washed in Tris-Buffered Saline (0.05M Tris, 0.15M NaCl, pH7.6):glycine (1.5% w/v in water) or in Tris-Buffered Saline alone, followed by a final rinse in water and drying (centrifuge- or air-dried). Alternatively, slides are immersed in 0.01 M Sodium Borohydride for 30 min, followed by extensive washes with water. Slides containing antibodies may be used immediately or can be stored at +4°C in a sealed, humidified chamber. Agarose slides (prepared as described above) are used for spotting of the affinity capture agent, as described for acrylamide gel pads above. Following spotting, slides are incubated in humidified sealed chambers at 4°C overnight. Unreacted active groups are blocked by incubation of slides in 1xTBS, 1xTBS/1.5%Glycine or 2M ethanolamine solution for at least one hour. Following the blocking step, the agarose slides are equilibrated in the binding buffer.

### b) Example of a non-covalent attachment technique

Silicon slides with polyacrylamide gel pads (for example obtained from Packard Bioscience, Meriden, Connecticut, USA) are treated with glutaraldehyde overnight, followed by 2% TFA for 15 min and extensively washed with water. Following that the slides are incubated with 0.01-1 mg/ml solutions of poly-Lysine (or poly-Arginine), followed by incubation with 0.01-1 mg/ml solution of poly-Glutamic acid (or poly-Aspartic acid), followed by incubation with 0.01-1 mg/ml solution of Protein A. Each incubation step above is followed by several washes with water washing steps. Prior to antibody spotting, the prepared slides can be stored at +4°C in a sealed,humidified chamber. Antibodies can be dispensed either using an arrayer (for example BioChip Arrayer with piezo-tips, Packard Bioscience, Meriden, Connecticut, USA) or by treating the whole or part of the slide with the solution containing the affinity capture agent. To block the remaining reactive sites, and to remove unbound antibodies, slides are washed in Tris-Buffered Saline (0.05M Tris, 0.15M NaCl, pH7.6):glycine (1.5% w/v in water) or in Tris-Buffered Saline alone, followed by a final rinse in H₂O and centrifuge-dried (or air dried). Slides containing, antibody are used immediately or can be stored at +4°C in a sealed, humidified chamber.

### c) Example of the combined attachment technique

Silicon slides containing, or coated with polyacrylamide gel pads (for example obtained from Packard Bioscience, Meriden, Connecticut, USA) are treated with glutaraldehyde overnight, followed by 2% TFA for 15 min and extensively washed with water. The slides then are incubated with a 0.01 -1 mg/ml solution of poly-Lysine, followed by a 0.01-1 mg/ml solution of poly-Glutamic acid (or poly-Aspartic acid), followed by a 0.01-1 mg/ml solution of Protein A. Each incubation step above is followed by extensive washes with water. Following Protein-A treatment, the slides are incubated with glutaraldehyde solution (1-10%) for 15-30 min and then washed with water. Affinity capture agents are spotted as described above, or, alternatively, the whole or part of the slide is incubated with the solution containing the affinity capture agent. To block the remaining reactive sites, and to remove unbound antibodies, slides are washed in water, followed by Tris-Buffered Saline (0.05M Tris, 0.15M NaCl, pH7.6). Slides containing antibody are used immediately or can be stored at +4°C in a sealed-humidified chamber.

### Example 2: Microarray of Antibodies

### 1. Glass/Silicon Surfaces

### a) Desorption of albumin from polyacrylamide pad

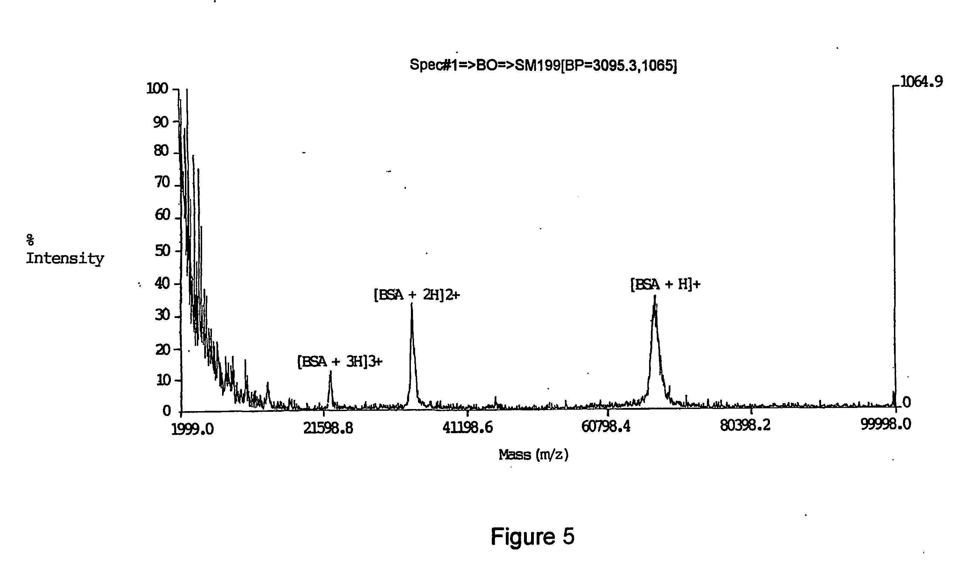

Bovine serum albumin (Sigma) (1 µl) was C18 cleaned using a Zip Tip^{™} (Millipore) and eluted with 0.5 ul of a 10 mg/ml solution of sinapinic acid in 70% acetonitrile. This was spotted directly onto polyacrylamide immobilized onto oxidized silicon. The silicon-polyacrylamide chip was placed in the modified target holder (figure 4). The spectrum in figure 5 was obtained using a Perseptive Voyager MALDI-MS in the linear mode and demonstrates the utility of silicon-immobilized polyacrylamide as a MALDI target suitable for proteins and peptides.

### b) Capture and desorption of EGF peptide from silicon

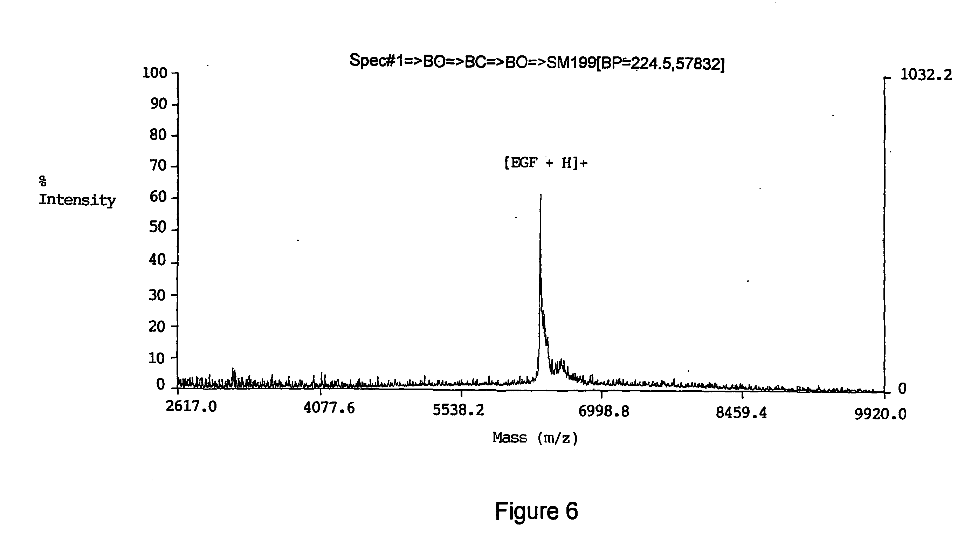

Oxidised silicon was silanized with 3-amino propyl silane, and activated by incubation under a glass coverslip with maleimide-succinimide, creating a thiol reactive surface. Anti-EGF antibody (Sigma - 1mg ml-1 in PBS) was spotted onto the reactive surface and allowed to dry. Unreacted maleimide groups were capped by incubating the whole silicon chip in 2-mercaptoethanol under a coverslip for 30 mins. 50ul of EGF peptide (Sigma -1 mg ml-1 in PBS) was placed onto the silicon under a coverslip and incubated at room temperature for 1 hour. Unbound EGF peptides was removed by washing the silicon chip 2 x 15 minutes in PBST, and 3 rinses in distilled water. The silicon chip was placed in the modifed target holder and 0.5 ul of sinapinic acid (10 mg/ml in 70% acetonitrile) was spotted on top of the previous spot and allowed to dry before being subjected to MALDI-MS in a Perseptive Voyager using the linear mode. Figure 6 shows the desorption/ionization of the EGF peptide, demonstrating the use of MALDI-MS to elute and characterize affinity bound antigens from immobilized antibodies.

### 2. Hydrogels

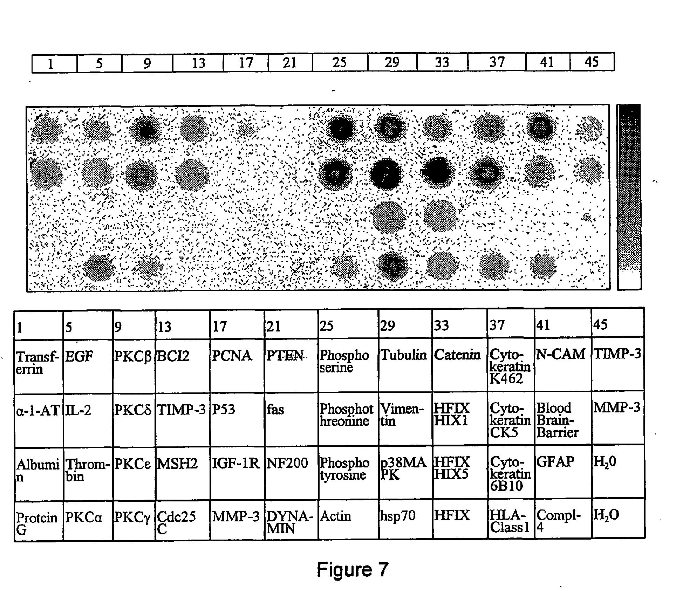

300pL of each of 45 antibodies (Sigma, CN Biosciences, see Table 1) at 0.1 mg ml-1 in PBS were arrayed onto hydrogel pads (Packard Biosciences) using a PiezoTip robot (Packard Biosciences) and allowed to dry. Unreacted aldehyde groups were reduced with sodium borohydride. The arrayed antibodies were incubated with 50µl (1 µg) of brain protein extract (Clontech) that had been labeled with Iodoacetamide-Cy5 (Oxford Glycosciences) in PBS for 3 hours under a coverslip. Unbound proteins were removed by 2 x 15 minute washes in PBST and 2 x rinses with distilled water. Fluorescent images were captured using a confocal laser scanner (ScanArray 3000 - Gsi lumonics). Each antibody has reproducibly generated a distinct level of fluorescence demonstrating the use of labeled protein and immobilized antibodies to generate quantitative signals proportional to protein abundance (Figure 7). Antibodies 34, 35, and 36 are specific to different parts of the human factor IX protein, demonstrating the variability in spot morphology and affinity inherent in antibody-antigen systems that do not utilize the invention described herein.

### Example 3: Characterization of Affinity Capture Agent

Affinity capture agents can be characterized and their specific antigenic determinants identified by contacting, under conditions which permit binding, with affinity reagents immobilized on a chip (Hydrogel on silicon slides) to a plurality of target peptide fragments, and using Mass Spectrometry to identify those target peptide fragments that are bound specifically to the immobilized affinity capture agents. This principle is exemplified by antibody capture of target peptide fragments generated from a complex tryptic digest of human serum albumin (HSA), followed by detection using MALDI-TOF-MS. This example also illustrates the ability of MS- based methods for scanning protein/ peptide arrays.

### 1. Affinity capture agent and incubation

In this example, a polyclonal anti-albumin antibody was used for peptide capture. The substrate used consisted of 2 mm diameter acrylamide based Hydrogel pads on a silicon slide (obtained from Packard). Prior to antibody immobilization, the slides were treated as follows:
1. Incubation in 10% glutaraldehyde for 4 hours, followed by a 30' wash with deionized water
2. Incubation in 2% TFA for 30', followed by a 20' wash with deionized water
3. Incubation in 0.4 mg/ml poly-Lysine (189 kDa) for 30', followed by a 20' water wash
4. Incubation in 0.4 mg/ml poly-Glutamic acid (31 kDa) for 40, followed by a 10' water wash
5. Incubation in 0.1 mg/ml solution of Protein-A for 1 hour, followed by a 19' water wash
6. Incubation in 10% glutaraldehyde for 15', followed by washing with water for 15'

Affinity capture agents were immobilized by immersing slides in a solution containing 0.01 mg/ml antibody in deionized water for 16 hours at 4°C, followed by a 30' wash with deionized water. Unreacted aldehyde groups were blocked using 1xTBS (2 hours at 4°C). Slides with immobilized antibody were rinsed with water and stored wet in sealed racks at 4°C.

### 2. Preparation of crude tryptic digest

Human Serum Albumin (HSA) was digested using modified porcine trypsin (Promega, Madison, WI). The HSA was reduced and alkylated prior to trypsinolysis in order that di-sulfide bonds would not form between cystein groups. Dithiothreitol (DTT, 10 mM in ammonium bicarbonate, 56 °C for 1 h) was used for the reduction step and the cysteine groups were then modified using iodoacetamide (55 mM in ammonium bicarbonate, 37 °C for 45 min). Typically 50 µg of HSA was digested with 1 ug trypsin (in 50 mM ammonium bicarbonate, pH 7.8). For two hours at 37 °C. In order to deactivate the trypsin prior to contacting with the array, the tryptic digest was boiled for 3 min in a water bath followed by addition of a protease inhibitor, phenylmethanesulphonlylfluoride (PMSF), to a final concentration of 1 mM. Incubation was done on slides with GeneFrame gaskets (ABgene, Surrey, UK) attached to them. 200 ul of binding buffer (1xTBS) containing 9 µg of digested HSA were applied to each GeneFrame (without cover slips) and incubation was carried for 15 hours at 4°C. Following incuabtion and prior to MS scanning, the slides were rinsed 10x times with deionized water, and further washed for 10' in water, 10' in 0.5xTBS, rinsed three times in water and further washed for 10' in waster.

### 3. Mass spectrometry

Arrays which have undergone incubation and washing steps were dried and prepared for MALDI-TOF MS. The MALDI technique requires a matrix which is absorbent at the wavelength of the desorption laser. The matrix used for the analysis of target peptides fragments was alpha cyano-4-hydroxycinnamic acid (CHCA). This was applied in two layers to the Hydrogel pads. Layer 1 (10 mg/ml CHCA in 60% acetonitrile) was applied using a hand pipette (<0.2 ul) and allowed to dry in air before application of layer 2 (0.2 ul of 7 mg/ml CHCA in 25% acetonitrile, 0.2% TFA). Once the second layer had dried, the chips were cut to fit a modified MALDI target. The spectra obtained were acquired using a Voyager DE-STR (Applied Biosystems, Framingham, MA) in the reflectron mode.

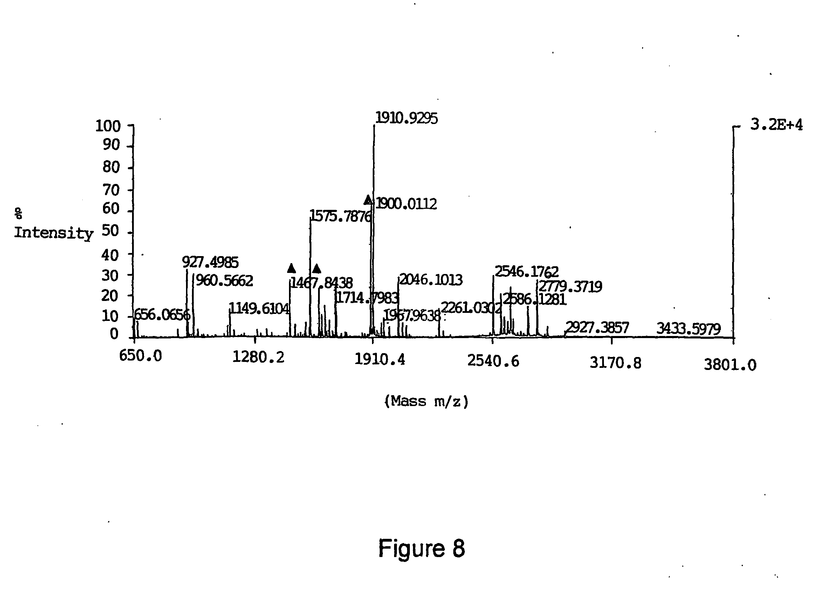

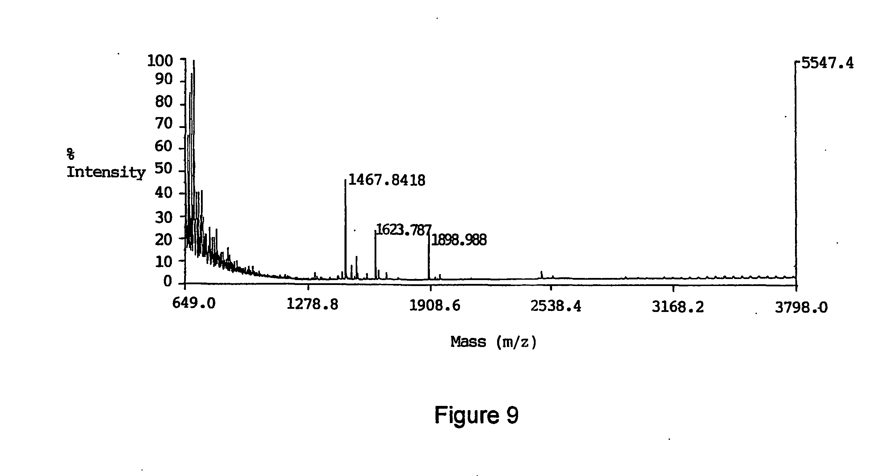

A MALDI-TOF MS spectrum of the crude tryptic digest of HSA that was used to contact the array under conditions that permit binding is shown in Figure 8. The digest used contained more than 100 target peptide fragments, some of which represented incompletely digested HSA (as checked using mass matching against the known in silico digested HSA). The MALDI-TOF spectrum obtained when the deactivated HSA digest was incubated with the immobilized polyclonal anti-albumin antibody is illustrated on Figure 9. The spectrum contains three peaks, which were present in the whole tryptic digest (denoted by triangles in Figure 8) and have been identified as HSA tryptic fragments from the known sequence:

**Table 4: HSA Tryptic Fragments**

| SEQ ID number | Peptide sequence of captured species from HSA tryptic digest | Actual mass to charge ratio | Detected mass to charge ratio (from figure 9) | Mass Accuracy (ppm) |
|---|---|---|---|---|
| 2 | RHPDYSVVLLLR | 1467.8436 | 1467.8418 | 0.3 |
| 3 | DVFLGMFLYEYAR | 1623.7881 | 1623.7877 | 0.3 |
| 4 | RHPYFYAPELLFFAK or HPYFYAPELLFFAKR | 1898.9958 | 1898.9687 | 1.3 |

The three specifically captured target peptide fragments, therefore, characterize the repertoire of interaction domains of the immobilized antibody.

### Example 4: Capture of Individual VCAM Peptides from Peptide Mixtures by scFv Antibodies.

### 1. Preparation of scFv array

Silicon slides containing polyacrylamide gel pads (e.g. Packard Bioscience, Meriden, CT, USA) were treated with glutaraldehyde (50%) solution overnight, washed in H₂O for 1 hour, and air-dried. A 100nl volume of each of four anti-VCAM peptide scFv antibodies (Cambridge Antibody Technologies) at 0.1 mg ml-1 in PBS were spotted onto 2 mm Hydrogel pads and incubated overnight at room temperature in a sealed humidified container. Polyacrylamide gel pad arrays were washed and unreacted aldehyde groups blocked in a 1 x TBS/glycine (1.5% w/v), [1:1] solution at room temperature for 2 hours.

### 2. Incubation of target peptide fragments with capture agent array

A 65µl sample containing 100ng of each of four VCAM peptides (K,B,M,J) in 1 x TBS buffer were incubated in sealed gaskets with identical anti- VCAM peptide scFv antibody arrays overnight at room temperature. Arrays were washed in 1 x TBS for 1 hour, H₂O for 20mins; 1 x TBS for 20mins and dried.

### 3. Mass spectrometry

Arrays of captured peptides were analysed by MALDI-TOF-MS exactly as described in the Example 3. MALDI-TOFmass spectra indicate that each scFv antibody captures their respective targets; no cross-reaction with unrelated peptides is noted (Figure 10).

The present invention is not to be limited in scope by the specific embodiments described herein. Indeed, various modifications of the invention in addition to those described herein will become apparent to those skilled in the art from the foregoing description and the accompanying figures.

It is further to be understood that all values are approximate, and are provided for description.

## Claims

1. A method for determining the presence of one or more proteins of interest in a sample, which method comprises:
a) submitting the sample to conditions that allow fragmentation of the proteins into target peptide fragments; and
b) contacting the target peptide fragments with an array of immobilized capture agents, the capture agents comprising those that are designed to recognize predicted target peptide fragments according to a chosen fragmentation protocol of the proteins of interest; whereby the binding of the target peptide fragments with the capture agents is indicative of the presence of the protein(s) in the sample.

2. The method according to claim 1, wherein the target peptide fragments are labeled.

3. The method according to claim 1 or claim 2, wherein the capture agents are antibodies.

4. The method according to any one of the preceding claims, which further comprises determining the relative amount of the proteins in the sample by quantitating the amount of the bound target peptide fragments.

5. The method according to claim 4, wherein the quantitation of the amount of the target peptide fragments comprises determining the amount relative to the amount of a peptide fragment of a constitutively expressed protein.

6. The method according to any one of the preceding claims, wherein step a) comprises contacting the sample with a proteolytic enzyme.

7. The method according to claim 6, wherein the proteolytic enzyme is an enzyme used to determine theoretical enzymatic cleavage of the protein.

8. The method according to claim 6, wherein the proteolytic enzyme is an enzyme used to obtain peptide fragments produced by enzymatic cleavage of the proteins.

9. The method according to any of claims 1-5, wherein step a) comprises contacting the sample with a chemical cleavage agent.

10. The method according to claim 9, wherein the chemical cleavage agent is used to determine theoretical chemical cleavage of the protein.

11. The method according to claim 9, wherein the chemical cleavage agent is used to obtain sequences of peptide fragments produced by chemical cleavage of the proteins.

12. The method according to any one of the preceding claims, which further comprises determining whether the target peptide fragments comprise a post-translational modification.

13. The method according to claim 12, wherein the determination of a post-translational modification comprises mass spectrum analysis.

14. The method according to any one of the preceding claim, wherein plural capture agents have similar affinity for their respective target peptide fragments to which they bind specifically.

15. The method according to claim 1, wherein the sample is pre-treated to obtain a protein preparation substantially free of contaminants.

16. The method according to claim 15, wherein the pre-treatment comprises one or more of fractionation, differential extraction of membrane or cytosolic fractions, selective depletion, application to an affinity column.

17. The method according to claim 1, wherein the sample is denatured prior to proteolysis.

18. The method according to claim 17, wherein the sample is denatured in 6 M guanidine HCl or 6-8 M urea, 50mM Tris-HCl (pH8), and 2-5mM DTT or 2-mercaptoethanol.

19. The method according to claim 1, wherein the sample is pre-treated with glycosidases to remove glycosylation side chains.

20. The method according to claim 1, wherein the sample is pre-treated to remove predictably varying post-translational modifications.

21. The method according to claim 1, wherein the sample is subjected to a reduction/alkylation step prior to proteolysis.

22. The method according to claim 1, wherein each of said capture agents is respectively highly specific for a unique sequence.

23. The method according to claim 1, wherein the array comprises two or more capture agents, each specific for a different target peptide fragment of a protein within the sample.

24. The method according to claim 1, wherein the array comprises two or more capture agents, each specific for a different target peptide fragment from the same or different proteins within the sample, wherein said two or more capture agents are put in the same spot on the array.

25. The method according to claim 24, wherein the binding of the target peptide fragments with the capture agents in b) is determined by MALDI-MS.

26. The method according to claim 1, wherein at least one target peptide fragment comprises a sequence unique to the protein being assayed.

27. The method according to claim 1, wherein at least one target peptide fragment is present in a region subject to post-translational modification.

## Patentansprüche

1. Verfahren zur Bestimmung der Gegenwart von einem oder mehr Protein(en) von Interesse in einer Probe, welches Verfahren Folgendes umfasst:
a) Aussetzen der Probe gegenüber Bedingungen, welche die Fragmentierung der Proteine zu Target-Peptidfragmenten zulassen; und
b) in Kontakt bringen der Target-Peptidfragmente mit einem Array immobilisierter Fänger-Agenzien, wobei die Fänger-Agenzien diejenigen umfassen, die zum Erkennen der vorhergesagten Target-Peptidfragmente gemäß einem gewählten Fragmentierungsprotokoll der Proteine von Interesse bestimmt sind; wobei die Bindung der Target-Peptidfragmente mit den Fänger-Agenzien auf das Vorliegen des Proteins/der Proteine in der Probe schließen lässt.

2. Verfahren nach Anspruch 1, worin die Target-Peptidfragmente markiert sind.

3. Verfahren nach Anspruch 1 order 2, worin die Fänger-Agenzien Antikörper darstellen.

4. Verfahren nach einem der vorangehenden Anspruche, das ferner die Bestimmung der relativen Menge der Proteine in der Probe durch Quantifizieren der Enge der gebundenen Target-Peptidfragmente umfasst.

5. Verfahren nach Anspruch 4, worin die Quantifizierung der Menge der Target-Peptidfragmente die Bestimmung der Menge relativ zur Menge eines Peptidfragments eines konstitutiv exprimierten Proteins umfasst.

6. Verfahren nach einem der vorangehenden Ansprüche, worin Schritt a) das in Kontakt bringen der Probe mit einem proteolytischen Enzym umfasst.

7. Verfahren nach Anspruch 6, worin das proteolytische Enzym ein Enzym darstellt, das zur Bestimmung der theoretischen enzymatischen Spaltung des Proteins verwendet wird.

8. Verfahren nach Anspruch 6, worin das proteolytische Enzym ein Enzym darstellt, das zum Erhalt von Peptidfragmenten verwendet wird, die durch enzymatische Spaltung der Proteine hergestellt werden.

9. Verfahren nach einem der Anspruche 1-5, worin Schritt a) das in Kontakt bringen der Probe mit einem chemischen Spaltmittel umfasst.

10. Verfahren nach Anspruch 9, worin das chemische Spaltmittel zur Bestimmung der theoretischen chemischen Spaltung des Proteins verwendet wird.

11. Verfahren nach Anspruch 9, worin das chemische Spaltmittel zum Erhalt von Peptidfragment-Sequenzen verwendet wird, die durch chemische Spaltung der Proteine hergestellt werden.

12. Verfahren nach einem der vorangehenden Ansprüche, das ferner die Bestimmung umfasst, ob die Target-Peptidfragmente eine posttranslationale Modifikation umfassen.

13. Verfahren nach Anspruch 12, worin die Bestimmung einer posttranslationalen Modifikation die Massenspektralanalyse umfasst.

14. Verfahren nach einem der vorangehenden Anspruch, worin mehrere Fänger-Agenzien eine ähnliche Affinität zu ihren entsprechenden Target-Peptidfragmenten aufweisen, an die sie spezifisch binden.

15. Verfahren nach Anspruch 1, worin die Probe zum Erhalt einer Proteinpräparation, die im Wesentlichen frei von Kontaminanten ist, vorbehandelt wird.

16. Verfahren nach Anspruch 15, worin die Vorbehandlung eines oder mehr von Folgendem umfasst: Fraktionierung, Differenzialextraktion von Membran- oder cytosolischen Fraktionen, selektive Depletion und Aufbringen auf eine Affinitätssäule.

17. Verfahren nach Anspruch 1, worin die Probe vor der Proteolyse denaturiert wird.

18. Verfahren nach Anspruch 17, worin die Probe in 6 M Guanidin-HCl oder 6-8 M Harnstoff, 50 mM Tris-HCl (pH 8) und 2-5 mM DTT oder 2-Mercaptoethanol denaturiert wird.

19. Verfahren nach Anspruch 1, worin die Probe zum Entfernen von Glycosylierungsseitenketten mit Glycosidasen vorbehandelt wird.

20. Verfahren nach Anspruch 1, worin die Probe zum Entfernen von vorhersagbar variierenden posttranslationalen Modifikationen vorbehandelt wird.

21. Verfahren nach Anspruch 1, worin die Probe vor der Proteolyse einem Reduktions-Alkylierungsschritt ausgesetzt wird.

22. Verfahren nach Anspruch 1, worin jedes der Fänger-Agenzien für eine einzigartige Sequenz entsprechend hochspezifisch ist.

23. Verfahren nach Anspruch 1, worin der Array zwei oder mehr Fänger-Agenzien umfasst, wobei jedes für ein verschiedenes Target-Peptidfragment eines Proteins in der Probe spezifisch ist.

24. Verfahren nach Anspruch 1, worin der Array zwei oder mehr Fänger-Agenzien umfasst, jedes spezifisch für ein verschiedenes Target-Peptidfragment aus den gleichen oder verschiedenen Proteinen in der Probe, worin die zwei oder mehr Fänger-Agenzien in den gleichen Fleck auf dem Array gegeben werden.

25. Verfahren nach Anspruch 24, worin die Bindung der Target-Peptidfragmente mit den Fänger-Agenzien in b) mittels MALDI-MS bestimmt wird.

26. Verfahren nach Anspruch 1, worin mindestens ein Target-Peptidfragment eine Sequenz umfasst, die für das zu bestimmende Protein einzigartig ist.

27. Verfahren nach Anspruch 1, worin mindestens ein Target-Peptidfragment in einer Region anwesend ist, die der posttranslationalen Modifikation ausgesetzt ist.

## Revendications

1. Procédé pour la détermination de la présence d'une ou de plusieurs protéines intéressantes dans un échantillon, lequel procédé comprend:
a) l'exposition de l'échantillon à des conditions qui permettent la fragmentation des protéines en fragments peptidiques cibles; et
b) la mise en contact des fragments peptidiques cibles avec un jeu ordonné d'agents de capture immobilisés, les agents de capture comprenant ceux qui sont conçus pour reconnaître des fragments peptidiques cibles prévus selon un protocole de fragmentation choisi des protéines intéressantes; en conséquence de quoi la liaison des fragments peptidiques cibles avec les agents de capture est indicatrice de la présence de la ou des protéines dans l'échantillon.

2. Procédé selon la revendication 1, dans lequel les fragments peptidiques cibles sont marqués.

3. Procédé selon la revendication 1 ou la revendication 2, dans lequel les agents de capture sont des anticorps.

4. Procédé selon l'une quelconque des revendications précédentes, qui comprend en outre la détermination de la quantité relative des protéines dans l'échantillon en quantifiant la quantité des fragments peptidiques cibles liés.

5. Procédé selon la revendication 4, dans lequel la quantification de la quantité des fragments peptidiques cibles comprend la détermination de la quantité relativement à la quantité d'un fragment peptidique d'une protéine exprimée constitutivement.

6. Procédé selon l'une quelconque des revendications précédentes, dans lequel l'étape a) comprend la mise en contact de l'échantillon avec une enzyme protéolytique.

7. Procédé selon la revendication 6, dans lequel l'enzyme protéolytique est une enzyme utilisée pour déterminer le clivage enzymatique théorique de la protéine.

8. Procédé selon la revendication 6, dans lequel l'enzyme protéolytique est une enzyme utilisée pour obtenir des fragments peptidiques produits par un clivage enzymatique des protéines.

9. Procédé selon l'une quelconque des revendications 1-5, dans lequel l'étape a) comprend la mise en contact de l'échantillon avec un agent de clivage chimique.

10. Procédé selon la revendication 9, dans lequel l'agent de clivage chimique est utilisé pour déterminer le clivage chimique théorique de la protéine.

11. Procédé selon la revendication 9, dans lequel l'agent de clivage chimique est utilisé pour obtenir des séquences de fragments peptidiques produits par un clivage chimique des protéines.

12. Procédé selon l'une quelconque des revendications précédentes, qui comprend en outre la détermination si les fragments peptidiques cibles comprennent une modification post-traductionnelle.

13. Procédé selon la revendication 12, dans lequel la détermination d'une modification post-traductionnelle comprend une analyse de spectre de masse.

14. Procédé selon l'une quelconque des revendications précédentes, dans lequel les agents de capture multiples présentent une affinité similaire pour leurs fragments peptidiques cibles respectifs auxquels ils se lient spécifiquement.

15. Procédé selon la revendication 1, dans lequel l'échantillon est traité au préalable pour obtenir une préparation de protéine substantiellement exempte de contaminants.

16. Procédé selon la revendication 15, dans lequel le traitement préalable comprend une ou plusieurs opérations parmi: un fractionnement, une extraction différentielle de fractions de membrane ou cytosotiques, un appauvrissement sélectif, une application sur une colonne d'affinité.

17. Procédé selon la revendication 1, dans lequel l'échantillon est dénaturé avant la protéolyse.

18. Procédé selon la revendication 17, dans lequel l'échantillon est dénaturé dans 6 M de guanidine HCl ou 6-8 M d'urée, 50 mM de Tris-HCl (pH 8) et 2-5 mM de DTT ou de 2-mercaptoéthanol.

19. Procédé selon la revendication 1, dans lequel l'échantillon est traité au préalable avec des glycosidases pour retirer les chaînes latérales de glycosylation.

20. Procédé selon la revendication 1, dans lequel l'échantillon est traité au préalable pour retirer des modifications post-traductionnelles variant de façon prévisible.

21. Procédé selon la revendication 1, dans lequel l'échantillon est soumis à une étape de réduction/alkylation avant la protéolyse.

22. Procédé selon la revendication 1, dans lequel chacun desdits agents de capture est respectivement hautement spécifique pour une séquence unique.

23. Procédé selon la revendication 1, dans lequel le jeu ordonné comprend deux agents de capture ou plus, chacun étant spécifique pour un fragment peptidique cible différent d'une protéine dans l'échantillon.

24. Procédé selon la revendication 1, dans lequel le jeu ordonné comprend deux agents de capture ou plus, chacun étant spécifique pour un fragment peptidique cible différent provenant de protéines identiques ou différents dans l'échantillon, dans lequel lesdits deux agents de capture ou plus sont placés dans le même point sur le jeu ordonné.

25. Procédé selon la revendication 24, dans lequel la liaison des fragments peptidiques cibles avec les agents de capture dans b) est déterminée par MALDI-MS.

26. Procédé selon la revendication 1, dans lequel au moins un fragment peptidique cible comprend une séquence unique pour la protéine qui est dosée.

27. Procédé selon la revendication 1, dans lequel au moins un fragment peptidique cible est présent dans une région donnant lieu à une modification post-traductionnelle.
